(19) Europäisches Patentamt
European Patent Office
Office européen des brevets

(11) Veröffentlichungsnummer: **0 045 964**
**A1**

(12) # EUROPÄISCHE PATENTANMELDUNG

(21) Anmeldenummer: **81106233.0**

(22) Anmeldetag: **10.08.81**

(51) Int. Cl.³: **C 07 C 103/26**
C 07 C 143/75, C 07 C 103/58
A 61 K 31/165, A 61 K 31/18

(30) Priorität: **13.08.80 CH 6118/80**

(43) Veröffentlichungstag der Anmeldung:
**17.02.82 Patentblatt 82/7**

(84) Benannte Vertragsstaaten:
**AT BE CH DE FR GB IT LI LU NL SE**

(71) Anmelder: **CIBA-GEIGY AG**
**Patentabteilung Postfach**
**CH-4002 Basel(CH)**

(72) Erfinder: **Ostermayer, Franz, Dr.**
**Am Hang 5**
**CH-4125 Riehen(CH)**

(72) Erfinder: **Zimmermann, Markus, Dr.**
**Steinbrecheweg 8**
**CH-4125 Riehen(CH)**

(74) Vertreter: **Zumstein sen., Fritz, Dr. et al,**
**Bräuhausstrasse 4**
**D-8000 München 2(DE)**

(54) **Derivate des 3-Amino-1,2-propandiols.**

(57) Derivate des 3-Amino-1,2-propandiols der Formel

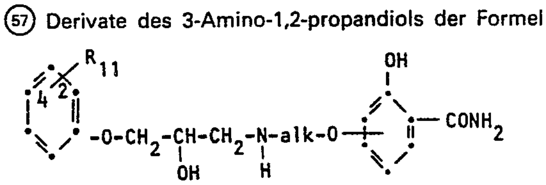

(1)

in welcher $R_{11}$ Polyhydroxyniederalkyl, Cycloalkylniederalkoxyniederalkyl, in 4-Stellung gebundenes Niederalkylsulfonylamino oder in 4-Stellung gebundenes 1-Niederalkylcarbamoyl-2-vinyl, und alk Reste der Formeln $-CH_2-CH_2-$ oder

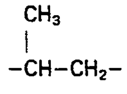

bedeutet, und Salze von solchen Verbindungen, Verfahren zu ihrer Herstellung, Arzneimittel, welche die neuen Verbindungen enthalten, und deren Verwendung als Blocker von adrenergen β-Rezeptoren zur Behandlung von Angina pectoris und Herzrythmusstörungen, sowie als blutdrucksenkende Mittel.

EP 0 045 964 A1

Croydon Printing Company Ltd.

CIBA-GEIGY AG
Basel (Schweiz)

4-13010/12253/+

Derivate des 3-Amino-1,2-propandiols.

Die Erfindung betrifft neue Derivate des 3-Amino-1,2-propandiols, Verfahren zu ihrer Herstellung, pharmazeutische Präparate, die solche Verbindungen enthalten und ihre Verwendung zur Herstellung von pharmazeutischen Präparaten oder als pharmakologisch wirksame Verbindungen.

Die erfindungsgemässen Verbindungen entsprechen der Formel

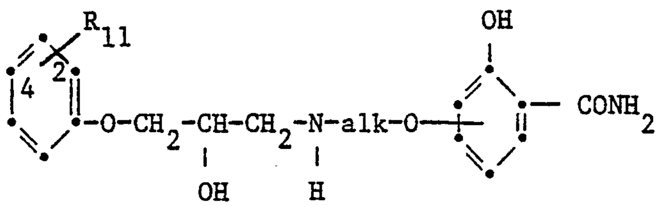

(I)

in welcher $R_{11}$ Polyhydroxyniederalkyl, Cycloalkylniederalkoxyniederalkyl, in 4-Stellung gebundenes Niederalkylsulfonylamino oder in 4-Stellung gebundenes 1-Niederalkylcarbamoyl-2-vinyl, und alk Reste der Formeln $-CH_2-CH_2-$ oder $-\overset{\overset{CH_3}{|}}{CH}-CH_2-$ bedeutet.

Zum Gegenstand der Erfindung gehören auch Salze solcher Verbindungen.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Cycloalkyl hat 3-7, insbesondere 3-5 Kohlenstoffatome als Ringglieder. Niederalkyl ist z.B. Methyl, Aethyl, n-Propyl, Isopropyl, n-Butyl, Isobutyl oder tert.-Butyl. Polyhydroxyniederalkyl kann im Phenylring jede der möglichen Stellungen einnehmen und ist z.B. Di- oder Trihydroxyniederalkyl, wie 1,2-Dihydroxy- oder 2,3-Dihydroxyniederalkyl, z.B. 1,2-Dihydroxy- oder 2,3-Dihydroxypropyl.

Cycloalkylniederalkoxyniederalkyl kann im Phenylring jede der möglichen Stellungen substituieren und ist z.B. Cycloalkylniederalkoxymethyl oder 1- oder 2-(Cycloalkylniederalkoxy)-äthyl. Niederalkylsulfonylamino entspricht der Formel Niederalkyl-$SO_2$-N($R_{12}$)-, worin $R_{12}$ für Wasserstoff oder Niederalkyl steht, und bedeutet dementsprechend z.B. Aethylsulfonylamino und insbesondere Methylsulfonylamino, ferner aber auch N-Methyl-methylsulfonylamino. 1-Niederalkylcarbamoyl-2-vinyl ist z.B. 1-Aethylcarbamoyl- und insbesondere 1-Methylcarbamoyl-2-vinyl.

Das die Amid- und die Hydroxygruppe tragende Phenyl ist mit dem restlichen Molekül in der Weise verbunden, dass letzteres in der 4-Stellung des genannten Phenyls, d.h. in der para-Stellung zur Amidgruppe, und vor allem in der 5-Stellung des genannten Phenyls, (d.h. in para-Stellung zur Hydroxygruppe) gebunden ist.

Die neuen Verbindungen können in Form ihrer Salze wie ihrer Säureadditionssalze und in erster Linie ihrer pharmazeutisch verwendbaren, nicht-toxischen Säureadditionssalze vorliegen. Geeignete Salze sind z.B. solche mit anorganischen Säuren, wie Halogenwasserstoffsäuren, z.B. Chlorwasserstoffsäure oder Bromwasserstoffsäure, Schwefelsäure, oder Phosphorsäure, oder mit organischen Säuren, wie aliphatischen, cycloaliphatischen, aromatischen oder heterocyclischen Carbon- oder Sulfonsäuren, z.B. Ameisen-, Essig-, Propion-, Bernstein-, Glykol-, Milch-, Aepfel-, Wein-, Zitronen-, Malein-, Hydroxymalein-, Brenztrauben-, Fumar-, Benzoe-, 4-Aminobenzoe-, Anthranil-, 4-Hydroxybenzoe-, Salicyl-, Embon-, Methansulfon-, Aethansulfon-, 2-Hydroxyäthansulfon-, Aethylensulfon-, Toluolsulfon-, Naphthalinsulfon- oder Sulfanilsäure, oder mit anderen sauren organischen Stoffen, wie Ascorbinsäure.

Die neuen Verbindungen besitzen wertvolle pharmakologische Eigenschaften. Insbesondere wirken sie in spezifischer Weise auf β-adrenerge Rezeptoren. Dieser Wirkung liegt als gemeinsame Eigenschaft

der Verbindungen der Formel I die Affinität zu diesen Rezeptoren zugrunde, die sich bei fehlender oder sehr geringer stimulierender Eigenwirkung als reine Blockade, bei geringer bis mässiger stimulierender Eigenwirkung als Blockade mit gleichzeitiger ISA, d.h. intrinsic sympathomimetric activity, äussert.

Die Verbindungen der Formel I zeigen eine blockierende Wirksamkeit auf adrenerge β-Rezeptoren mit ausgeprägter Cardioselektivität, die sich insbesondere bei solchen Substanzen feststellen lässt, die in 4-Stellung des Phenylringes durch Niederalkylsulfonylamino, z.B. Methylsulfonylamino, oder durch 1-Niederalkylcarbamoyl-2-vinyl, z.B. 1-Methylcarbamoyl-2-vinyl, substituiert sind, während solche Substanzen, deren Phenylring durch Cycloalkylniederalkoxy-niederalkyl, z.B. Cyclopropylmethoxyäthyl, substituiert ist, eine weniger ausgeprägte Cardioselektivität aufweisen. Die Verbindungen der Formel I bewirken ferner eine Blockade von vasculären β-Rezeptoren, die sich insbesondere bei den Substanzen feststellen lässt, deren Phenylring durch Polyhydroxyniederalkyl, z.B. 2,3-Dihydroxypropyl, oder durch Cycloalkylniederalkoxy-niederalkyl, etwa Cyclopropylmethoxyäthyl, substituiert ist, wobei sowohl bei diesen Verbindungen aber auch bei denjenigen, die in 4-Stellung des Phenylringes eine Niederalkylsulfonylamino-, z.B. eine Methylsulfonylaminogruppe, oder eine 1-Niederalkylcarbamoyl-2-vinyl-, etwa eine 1-Methylcarbamoyl-2-vinylgruppe tragen, als zusätzliche Wirkung eine mehr oder weniger ausgeprägte Blockade trachealer β-Rezeptoren vorhanden ist.

Verbindungen der Formel I mit einer Polyhydroxyniederalkyl-, etwa einer 2,3-Dihydroxypropylgruppe, zeigen ausserdem eine mehr oder weniger ausgeprägte ISA, wobei als zusätzliche Wirkung eine Blutdrucksenkung zu nennen ist. Diese letzere Wirksamkeit lässt sich auch an Verbindungen mit einer Niederalkylsulfonylamino-, etwa einer Methylsulfonylaminogruppe im Phenylring, nachweisen.

Die vorstehenden Angaben betreffend pharmakologische Eigenschaften beruhen auf den Resultaten von entsprechenden pharmakologischen Versuchen in üblichen Testverfahren. So zeigen die neuen β-blockierenden Verbindungen eine Hemmung der durch Isoproterenol induzierten Tachykardie am isolierten Meerschweinchenherzen in einem Konzentrationsbereich von etwa 0,001 μg/ml bis etwa 3 μg/ml, und an der narkotisierten, mit Reserpin vorbehandelten Katze in einem Dosenbereich von etwa 0,001 mg/kg bis etwa 10 mg/kg bei intravenöser Verabreichung. Die Blockade von vasculären β-Rezeptoren ist durch die Hemmung der durch Isoproterenol induzierten Vasodilatation im Gefässgebiet der A. femoralis an der narkotisierten, mit Reserpin vorbehandelten Katze bei intravenöser Verabreichung in einem Dosenbereich von etwa 0,003 mg/kg bis etwa 30 mg/kg nachweisbar.

Die durch die Verbindungen der Formel I bewirkte Blockade der trachealen β-Rezeptoren lässt sich mittels der Hemmung der durch Isoprenalin induzierten Relaxation an der isolierten Meerschweichentrachea in einem Konzentrationsbereich von etwa 0,001 μ Mol/Liter bis etwa 30 μ Mol/Liter nachweisen.

Die ISA der β-blockierenden Verbindungen der Formel I ergibt sich aus der Zunahme der basalen Herzfrequenz an der narkotisierten, mit Reserpin vorbehandelten Katze, was sich z.B. bei intravenöser Verabreichung von 1-[2-(3-Carbamoyl-4-hydroxy-phenoxy)-äthylamino]-3-[2-(2,3-dihydroxypropyl)-phenoxy]-2-propanol in einem Dosenbereich von etwa 0,001 mg/kg bis etwa 1 mg/kg zeigen lässt.

Die neuen β-blockierenden Verbindungen bewirken ferner in einem Dosenbereich von etwa 0,03 mg/kg bis etwa 30 mg/kg i.v. eine Senkung des arteriellen Blutdrucks bei der narkotisierten Katze.

Die neuen Verbindungen der Formel I können demnach als, teilweise cardioselektiv wirksame, β-Rezeptoren-Blocker z.B. zur Behandlung von Herzrythmusstörungen, Angina pectoris sowie der Hypertonie verwendet werden.

Die Verbindungen der allgemeinen Formel I können auch als wertvolle Zwischenprodukte für die Herstellung anderer wertvoller, insbesondere pharmazeutisch wirksamer, Verbindungen verwendet werden.

Die Erfindung betrifft insbesondere Verbindungen der Formel I, worin $R_{11}$ Di- oder Trihydroxyniederalkyl, Cycloalkylniederalkoxyniederalkyl mit bis zu 4 Kohlenstoffatomen im Niederalkylteil, in 4-Stellung gebundenes Niederalkylsulfonylamino- oder N-Niederalkyl-niederalkylsulfonylamino mit bis zu 4 Kohlenstoffatomen in jedem Niederalkylteil, oder in 4-Stellung gebundenes 1-Niederalkylcarbamoyl-2-vinyl mit bis zu 4 Kohlenstoffatomen im Niederalkylteil und alk Reste der Formeln $-CH_2-CH_2-$ oder $-CH(CH_3)-CH_2-$ bedeutet, oder Salze, insbesondere Säureadditionssalze, vor allem pharmazeutisch verwendbare nicht-toxische Säureadditionssalze davon.

Die Erfindung betrifft insbesondere Verbindungen der Formel I, worin $R_{11}$ 1,2-Dihydroxy- oder 2,3-Dihydroxyniederalkyl, Cycloalkylniederalkoxymethyl oder 1- oder 2-(Cycloalkylniederalkoxy)-äthyl, in 4-Stellung gebundenes Niederalkylsulfonylamino mit bis zu 4 Kohlenstoffatomen, wobei $R_{12}$ Wasserstoff ist, oder in 4-Stellung gebundenes 1-Aethylcarbamoyl-2-vinyl oder 1-Methylcarbamoyl-2-vinyl und alk Reste der Formeln $-CH_2-CH_2-$ oder $-CH(CH_3)-CH_2-$ bedeutet, oder Salze, insbesondere Säureadditionssalze, vor allem pharmazeutisch verwendbare nicht-toxische Säureadditionssalze davon.

Die Erfindung betrifft insbesondere Verbindungen der Formel I, worin $R_{11}$ 1,2-Dihydroxy- oder 2,3-Dihydroxyniederalkyl mit bis zu 4 Kohlenstoffatomen, z.B. 1,2-Dihydroxy- oder 2,3-Dihydroxypropyl, 1- oder 2-(Cycloalkylniederalkoxy)-äthyl mit 3-5 Kohlenstoffatomen im Cycloalkylteil, z.B. 1- oder 2-(Cyclopropylmethoxy)-äthyl, in 4-Stellung gebundenes Niederalkylsulfonylamino mit bis zu 4 Kohlenstoffatomen, wobei $R_{12}$ Wasserstoff ist, z.B. Methylsulfonylamino, oder 1-Methylcarbamoyl-2-vinyl, und alk Reste der Formeln $-CH_2-CH_2-$ oder $-CH(CH_3)-CH_2-$

bedeutet, oder Salze, insbesondere Säureadditionssalze, vor allem pharmazeutisch verwendbare, nicht toxische Säureadditionssalze davon.

Die neuen Verbindungen der Formel I werden in an sich bekannter Weise hergestellt. Man kann sie z.B. erhalten, indem man eine Verbindung der Formel

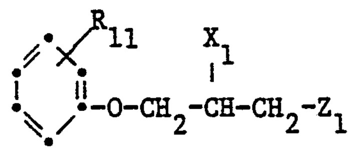

(II)

mit einer Verbindung der Formel

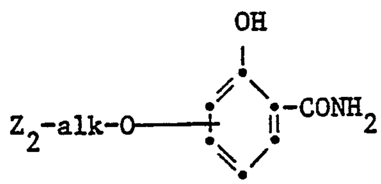

(III),

worin eine der Gruppen $Z_1$ und $Z_2$ eine reaktionsfähige veresterte Hydroxygruppe darstellt und die andere für die primäre Aminogruppe steht und $X_1$ Hydroxy bedeutet, oder worin $X_1$ und $Z_1$ zusammen die Epoxygruppe bedeuten und $Z_2$ für die primäre Aminogruppe steht, umsetzt, und , wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren oder ein erhaltenes Racemat in die Antipoden auftrennt.

Eine reaktionsfähige veresterte Hydroxygruppe $Z_1$ bzw. $Z_2$ ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die obige Reaktion wird in an sich bekannter Weise durchgeführt, wobei man, besonders bei Verwendung eines Ausgangsmaterials mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetallniederalkanolats, und/oder eines Ueberschusses des basischen Reaktionsteilnehmers und üblicherweise in Gegenwart eines Lösungsmittels oder Lösungsmittelgemisches und, wenn notwendig, unter Kühlen oder Erwärmen, z.B. in einem Temperaturbereich von etwa -20°C bis etwa +150°C, in einem offenen oder geschlossenen Gefäss und/oder in einer Inertgasatmosphäre, z.B. in einer Stickstoffatmosphäre, arbeitet.

Ausgangsstoffe der Formel II sind bekannt oder können in an sich bekannter Weise hergestellt werden.

Ausgangsstoffe der Formel III können z.B. durch Umsetzung eines Hydroxysalicylamids mit einem der Bedeutung von alk entsprechenden Dihalogenalkan, etwa einem Chlorbrom- oder Dibromalkan in Gegenwart eines alkalischen Kondensationsmittels, wie einem Alkalicarbonat, erhalten werden. Diese Umsetzungen werden in üblicher Weise vorgenommen, wobei an den Hydroxygruppen stehende Schutzgruppen gleichzeitig oder, wie nachfolgend beschrieben, anschliessend abgespalten werden.

Die Verbindungen der Formel I können weiterhin hergestellt werden, indem man in einer Verbindung der Formel

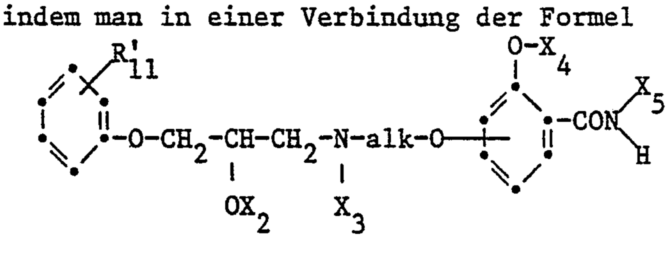

(IV),

worin $R'_{11}$ die Bedeutung von $R_{11}$ hat, oder in 4-Stellung gebundenes, am Stickstoff durch einen durch Wasserstoff ersetzbaren Substituenten substituiertes Niederalkylsulfonylamino darstellt, $X_2$, $X_3$, $X_4$ und $X_5$ jeweils Wasserstoff oder einen durch Wasserstoff ersetzbaren Substituenten bedeuten oder $X_2$ und $X_3$ und/oder $X_4$ und $X_5$ zusammen einen zweiwertigen, durch zwei Wasserstoffatome ersetzbaren Rest bedeuten, mit der Massgabe, dass mindestens einer der Reste $X_2$, $X_3$, $X_4$ und $X_5$ von Wasserstoff verschieden ist, oder mindestens $R'_{11}$ in 4-Stellung gebundenes, am Stickstoffatom durch einen durch Wasserstoff ersetzbaren Substituenten substituiertes Niederalkylsulfonylamino darstellt, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon, die von Wasserstoff verschiedenen Gruppen $X_2$, $X_3$, $X_4$ und $X_5$, bzw. $X_2$ und $X_3$ zusammen und/oder $X_4$ und $X_5$ zusammen abspaltet und durch Wasserstoffatome ersetzt, und/oder einen am Stickstoffatom von in 4-Stellung gebundenem Niederalkylsulfonylamino $R'_{11}$ stehenden durch Wasserstoff ersetzbaren Substituenten durch Wasserstoff ersetzt, und, wenn erwünscht, die anschliessend an das erste Verfahren genannten zusätzlichen Verfahrensschritte durchführt.

Die Abspaltung der Gruppen $X_2$, $X_3$, $X_4$ oder $X_5$ oder jeweils $X_2$ und $X_3$ und/oder $X_4$ und $X_5$ zusammen, sowie des in einer 4-Niederalkylsulfonylaminogruppe am Stickstoffatom stehenden Substituenten wird mittels Solvolyse, wie Hydrolyse, Alkoholyse oder Acidolyse, oder mittels Reduktion, einschliesslich Hydrogenolyse vorgenommen.

Eine besonders geeignete abspaltbare Gruppe $X_3$ oder $X_4$ oder eine in einer 4-Niederalkylsulfonylaminogruppe am Stickstoffatom gebundene Schutzgruppe ist in erster Linie eine hydrogenolytisch abspaltbare $\alpha$-Arylniederalkylgruppe, wie eine gegebenenfalls substituierte 1-Polyphenylniederalkyl- oder 1-Phenylniederalkylgruppe, worin Substituenten, insbesondere des Phenylteils, z.B. Niederalkyl, wie Methyl, oder Niederalkoxy wie Methoxy sein können, und in erster Linie

Benzyl. Eine Gruppe $X_3$ und insbesondere $X_2$ und $X_4$ sowie eine Stickstoff-Schutzgruppe in einer 4-Niederalkylsulfonylaminogruppe können auch einen solvolytisch, wie hydrolytisch oder acidolytisch, ferner einen reduktiv, einschliesslich hydrogenolytisch, abspaltbaren Rest, insbesondere einen entsprechenden Acylrest, wie den Acylrest einer organischen Carbonsäure, z.B. Niederalkanoyl, wie Acetyl, oder Aroyl, wie Benzoyl, ferner den Acylrest eines Halbesters der Kohlensäure, wie Niederalkoxycarbonyl, z.B. Methoxycarbonyl, Aethoxycarbonyl oder tert.-Butyloxycarbonyl, 2-Halogenniederalkoxycarbonyl, z.B. 2,2,2-Trichloräthoxycarbonyl oder 2-Jodäthoxycarbonyl, gegebenenfalls substituiertes 1-Phenylniederalkoxycarbonyl, z.B. Benzyloxycarbonyl oder Diphenylmethoxycarbonyl, oder Aroylmethoxycarbonyl, z.B. Phenacyloxycarbonyl, ferner eine gegebenenfalls substituierte 1-Polyphenylniederalkylgruppe, worin Substituenten, in erster Linie des Phenylteils, z.B. die oben gegebene Bedeutung haben, und in erster Linie Trityl darstellen.

Ein durch $X_2$ und $X_3$ und/oder $X_4$ und $X_5$ zusammen gebildeter, abspaltbarer Rest ist in erster Linie eine hydrogenolytisch abspaltbare Gruppe, wie gegebenenfalls substituiertes 1-Phenyl-niederalkyliden, worin Substituenten, insbesondere des Phenylteils, z.B. Niederalkyl oder Niederalkoxy sein können, und insbesondere Benzyliden, sowie solvolytisch, insbesondere hydrolytisch spaltbare Gruppen, wie Niederalkyliden, z.B. Methylen oder Isopropyliden, oder 1-Phenylniederalkyliden, dessen Phenylteil gegebenenfalls durch Niederalkyl, wie Methyl oder Niederalkoxy, wie Methoxy, substituiert ist, insbesondere Benzyliden, oder Cycloalkyliden, z.B. Cyclopentyliden oder Cyclohexyliden.

In der Form von Salzen verwendbare Ausgangsstoffe werden in erster Linie in der Form von Säureadditionssalzen, z.B. mit Mineralsäuren, sowie mit organischen Säuren verwendet.

Hydrogenolytisch abspaltbare Reste $X_2$, $X_3$, $X_4$ und/oder $X_5$, insbesondere gegebenenfalls substituierte 1-Phenylniederalkylgruppen, ferner auch geeignete Acylgruppen, wie gegebenenfalls substituiertes 1-Phenylniederalkoxycarbonyl, sowie durch die Gruppen $X_2$ und $X_3$ sowie $X_4$ und $X_5$ zusammen gebildete, gegebenenfalls substituierte 1-Phenylniederalkylidengruppen, sowie in einer 4-Niederalkylsulfonylaminogruppe am Stickstoff stehende Schutzgruppen dieser Art können durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

Hydrolytisch abspaltbare Gruppen $X_2$, $X_3$, $X_4$ und/oder $X_5$, wie Acylreste von organischen Carbonsäuren, z.B. Niederalkanoyl, und von Halbestern der Kohlensäure, z.B. Niederalkoxycarbonyl, ferner z.B. Tritylreste, sowie durch die Reste $X_2$ und $X_3$ und/oder $X_4$ und $X_5$ zusammen gebildete Niederalkyliden-, 1-Phenyl-niederalkyliden- oder Cycloalkylidengruppen sowie die in einer 4-Niederalkylsulfonylaminogruppe vorhandene Stickstoff-Schutzgruppe dieser Art können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats oder eines Amins, wie Isopropylamin, abgespalten werden.

Acidolytisch abspaltbare Reste $X_2$, $X_3$, $X_4$ und/oder $X_5$ und/oder eine in einer 4-Niederalkylsulfonylaminogruppe vorhandene Stickstoff-Schutzgruppe sind insbesondere gewisse Acylreste von Halbestern der Kohlensäure, wie z.B. tert.-Niederalkoxycarbonyl oder gegebenenfalls substituierte Diphenylmethoxycarbonylreste, ferner auch der tert.-Niederalkylrest; solche Reste können durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden.

Unter reduktiv abspaltbaren Resten $X_2$, $X_3$, $X_4$ und/oder $X_5$ und/oder eine Stickstoff-Schutzgruppe in einer 4-Niederalkylsulfonylaminogruppe werden auch solche Gruppen verstanden, die beim Behandeln mit einem chemischen Reduktionsmittel (insbesondere mit einem reduzierenden Metall oder einer reduzierenden Metallverbindung) abgespalten werden. Solche Reste sind insbesondere 2-Halogenniederalkoxycarbonyl oder Arylmethoxycarbonyl, die z.B. beim Behandeln mit einem reduzierenden Schwermetall, wie Zink, oder mit einem reduzierenden Schwermetallsalz, wie einem Chrom(II)salz, z.B. -chlorid oder -acetat, üblicherweise in Gegenwart einer organischen Carbonsäure, wie Ameisensäure oder Essigsäure, und von Wasser abgespalten werden können.

Stickstoff-Schutzgruppen welche am Stickstoffatom einer 4-Niederalkylsulfonylaminogruppe $R'_{11}$ stehen, entsprechen den vorhin genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Die obigen Reaktionen werden üblicherweise in Gegenwart eines Lösungsmittels, oder Lösungsmittelgemisches durchgeführt, wobei geeignete Reaktionsteilnehmer gleichzeitig auch als solche funktionieren können, und, wenn notwendig, unter Kühlen oder Erwärmen, z.B. in einem offenen oder geschlossenen Gefäss und/oder in der Atmosphäre eines Inertgases, z.B. Stickstoff.

Die Ausgangsstoffe der Formel IV lassen sich analog den oben beschriebenen Verfahrensmodifikationen erhalten, z.B. durch Behandeln einer Verbindung der Formel

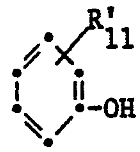

oder einem Salz davon, mit einer Verbindung

der Formel

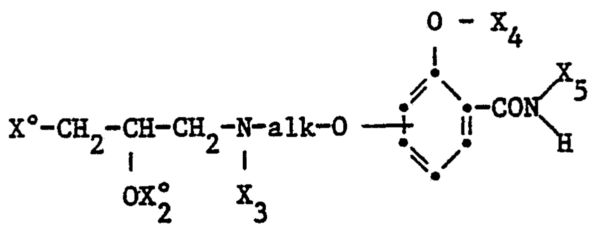

(IVa)

worin $X_2^\circ$ für die Gruppe $X_2$ steht, wobei mindestens eine der Gruppen $X_3$ und $X_2^\circ$ von Wasserstoff verschieden ist, und X° eine reaktionsfähige veresterte Hydroxygruppe bedeutet, oder $X_2^\circ$ und X° zusammen eine Kohlenstoff-Sauerstoff-Bindung darstellen, oder worin $X_3$ und $X_2^\circ$ zusammen einen abspaltbaren, durch zwei, mit dem Sauerstoff- bzw. Stickstoffatom verbundene Wasserstoffatome ersetzbaren Rest darstellen und X° eine reaktionsfähige veresterte Hydroxygruppe bedeutet, oder durch Behandeln einer Verbindung der Formel

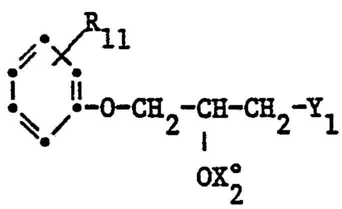

(IVb)

mit einer Verbindung der Formel

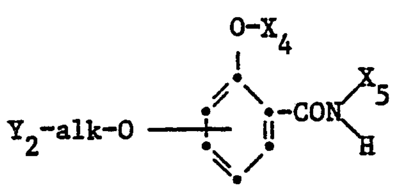

(IVc)

worin $X_2^\circ$ die oben für $X_2$ angegebene Bedeutung hat, und eine der Gruppen $Y_1$ und $Y_2$ eine reaktionsfähige veresterte Hydroxygruppe darstellt, und die andere für die Gruppe der Formel $-NH(X_3)$ steht, worin $X_3$ die oben gegebene Bedeutung hat, mit der Massgabe, dass mindestens

eine der Gruppen $X_3$ und $X_2$ von Wasserstoff verschieden ist, oder worin $X_2^o$ und $Y_1$ eine Sauerstoff-Kohlenstoff-Bindung bilden und $Y_2$ für die Gruppe der Formel $-NH(X_3)$ steht und $X_3$ von Wasserstoff verschieden ist. Die obigen Reaktionen werden in an sich bekannter Weise, z.B. wie unter dem ersten erfindungsgemässen Verfahren beschrieben, durchgeführt.

Man kann ferner z.B. die durch Umsetzung einer Verbindung der Formel

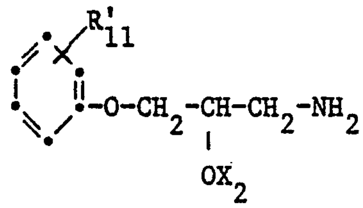

(IVd)

mit einer Carbonylverbindung der Formel

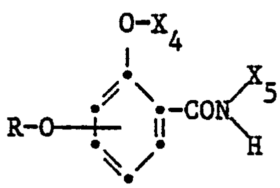

(IVe),

worin R einen dem Alkylenrest alk entsprechenden, eine vom Sauerstoffatom durch ein Kohlenstoffatom getrennte Carbonylgruppierung enthaltenden Alkylrest bedeutet, und $X_4$ und $X_5$ oder $X_4$ und $X_5$ zusammen eine der angegebenen Schutzgruppen bedeuten, gebildete Schiff'sche Base mit einem Borhydrid, etwa Natriumborhydrid zur Verbindung der Formel IV reduzieren. Die Reduktion kann auch mittels aktiviertem Wasserstoff in Gegenwart eines Hydrierkatalysators, z.B. eines Platin-auf-Kohle Katalysators erfolgen.

Carbonylverbindungen der Formel (IVe) wiederum können durch Umsetzung einer Verbindung der Formel

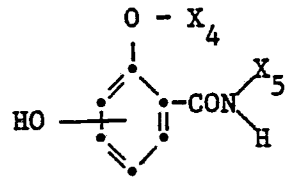

(IVf)

mit einer Verbindung der Formel R-Hal (IVg), worin R obige Bedeutung hat und z.B. ein Halogenketon, etwa Chloraceton darstellt, in üblicher Weise erhalten werden.

Die neuen Verbindungen der Formel I können ebenfalls erhalten werden, indem man in einer Verbindung der Formel

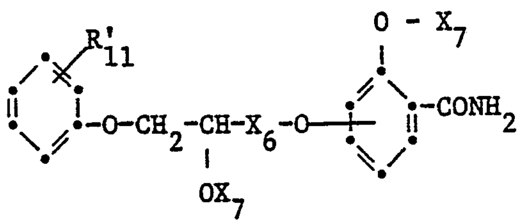

(V),

worin $X_6$ eine reduzierbare Gruppe der Formeln

$-CH = N - alk -$ (Va), bzw.

$-CH_2 - N = alk_1 -$ (Vb)

ist, wobei $alk_1$ für einen, dem Rest alk entsprechenden Alkylylidenrest steht und $X_7$ Wasserstoff oder einen unter den Bedingungen für die Reduktion von $X_6$ durch Wasserstoff ersetzbaren Rest darstellt, $R'_{11}$ die Bedeutung von $R_{11}$ hat, oder in 4-Stellung gebundenes, am Stickstoffatom durch einen durch Wasserstoff ersetzbaren Substituenten $X_7$ substituiertes Niederalkylsulfonylamino darstellt, worin $X_7$ die vorstehend angegebene Bedeutung hat, wobei stets $X_6$ eine reduzierbare Gruppe Va oder Vb ist, diese Gruppe reduziert und gleichzeitig die von Wasserstoff verschiedene Gruppe $X_7$ abspaltet und durch Wasserstoff ersetzt, und, wenn erwünscht, die anschliessend an das erste Verfahren genannten zusätzlichen Verfahrensschritte durchführt.

Eine hydrogenolytisch abspaltbare Gruppe $X_7$ ist in erster Linie eine α-Arylniederalkylgruppe, wie eine gegebenenfalls substituierte 1-Phenylniederalkylgruppe, worin Substituenten des Phenylteils, z.B. Niederalkoxy, wie Methoxy sein können, und ganz besonders Benzyl.

Stickstoff-Schutzgruppen $X_7$ in einer 4-Niederalkylsulfonylaminogruppe $R'_{11}$ entsprechen den vorhin für $X_7$ genannten und mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Ausgangsstoffe der Formel V mit einer Gruppe $X_6$ der Formel Vb können auch in der isomeren Form von Ring-Tautomeren der Formel

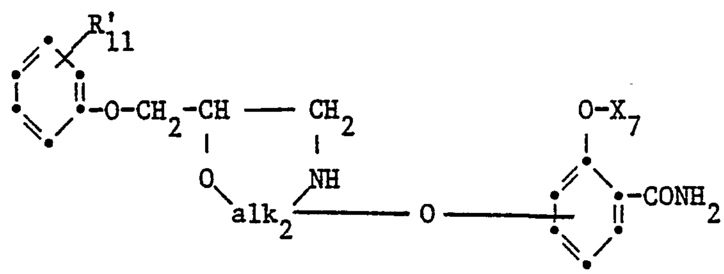

(Vd)

worin $alk_2$ der Bedeutung von $alk_1$ entspricht und das Sauerstoff- und Stickstoffatom des Ringes an das gleiche Kohlenstoffatom gebunden sind, vorliegen.

Eine Alkyl-ylidengruppe $alk_1$ ist z.B. Methin oder Aethyl-yliden, während eine Alkylidengruppe $alk_2$ z.B. Methylen, Aethyliden oder 1-Methyl-äthyliden darstellt.

Die Reduktion der Stickstoff-Kohlenstoff-Doppelbindung in Ausgangsstoffen der Formel V, die als $X_6$ eine Gruppe Va oder Vb enthalten, während $X_7$ die unter der Formel V angegebene Bedeutung hat, zur Stickstoff-Kohlenstoff-Einfachbindung kann in an sich bekannter Weise, z.B. durch Behandeln mit katalytisch aktiviertem Wasserstoff, wie

Wasserstoff in Gegenwart eines geeigneten Hydrierkatalysators, z.B. eines Nickel-, Platin- oder Palladiumkatalysators, erfolgen, wobei hydrogenolytisch abspaltbare Gruppen $X_7$ zugleich abgespalten und durch Wasserstoff ersetzt werden; oder man arbeitet mit einem geeigneten Hydridreduktionsmittel, wie einem Alkalimetallborhydrid, z.B. Natriumborhydrid. Bei Anwendung von Hydridreduktionsmitteln, können auch an Sauerstoff gebundene Acylreste von Carbonsäuren, wie z.B. der Essigsäure, als Reste $X_7$ vorliegen und im gleichen Arbeitsgang abgespalten werden.

Ein Ausgangsmaterial der Formel V kann in an sich bekannter Weise, gegebenenfalls in situ, d.h. unter den Bedingungen des beschriebenen Verfahrens, hergestellt werden. So kann man eine Verbindung der Formel

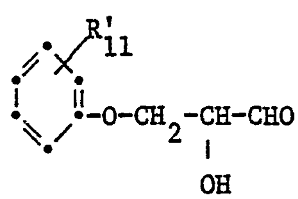

(Ve)

mit einem Amin der Formel

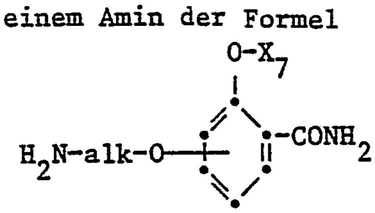

(Vf)

zu einem Ausgangsprodukt der Formel V mit der Gruppe $X_6$ der Formel Va umsetzen.

Durch Umsetzung einer Verbindung der Formel

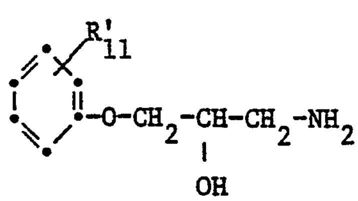

(Vh)

mit einer Carbonylverbindung der Formel

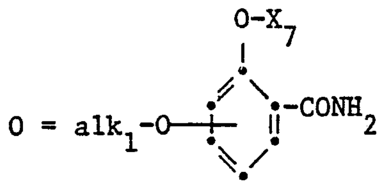

(Vi)

kann man zu Ausgangsstoffen der Formel V mit einer Gruppe $X_6$ der Formel (Vb) gelangen. Eine Modifizierung dieser Umsetzungen besteht darin, dass man eine einer Verbindung der Formel (Vh) entsprechende Dibenzylaminoverbindung, mit der Oxoverbindung der Formel (Vi) unter den reduzierenden Bedingungen des Verfahrens umsetzt. Hierbei verwendet man als Reduktionsmittel in erster Linie katalytisch aktivierten Wasserstoff, z.B. Wasserstoff in Gegenwart eines Schwermetallhydrierkatalysators oder eines Gemisches davon, wie eines Palladium- und/oder Platinkatalysators. Unter solchen Reaktionsbedingungen werden hydrogenolytisch abspaltbare Gruppen $X_7$, z.B. Benzylgruppen, abgespalten, und gleichzeitig die Stickstoff-Kohlenstoff-Doppelbindung zur entsprechenden Stickstoff-Kohlenstoff-Einfachbindung reduziert.

Oxoverbindungen der Formel (Vi) wiederum, sind z.B. durch Umsetzung einer Verbindung der Formel

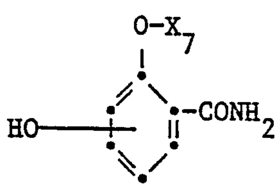

(Vk)

mit einer Halogenalkanon-Verbindung der oben erläuterten Formel R - Hal (IVf), z.B. Chloraceton, in Gegenwart eines alkalischen Kondensationsmittels, etwa Kaliumcarbonat, oder einer organischen Base, wie Triäthylamin, erhältlich.

Die neuen Verbindungen der Formel I können ebenfalls erhalten werden, indem man eine Verbindung der Formel

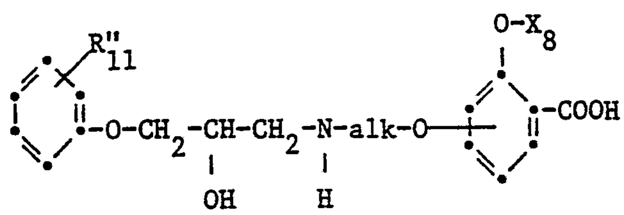

(VI),

worin $R''_{11}$ die Bedeutung von $R_{11}$ hat, oder in 4-Stellung gebundenes, gegebenenfalls am Stickstoffatom durch einen mittels Ammonolyse abspaltbaren und durch einen durch Wasserstoff ersetzbaren Substituenten substituiertes Niederalkylsulfonylamino darstellt, $X_8$ Wasserstoff oder eine mittels Ammonolyse abspaltbare Gruppe bedeutet, oder ein reaktionsfähiges Derivat einer der in Formel VI definierten Carbonsäuren, mit Ammoniak (VII) umsetzt, und gleichzeitig gegebenenfalls vorhandene Reste $X_8$ oder am Stickstoff einer 4-Niederalkylsulfonylaminogruppe stehende Substituenten abspaltet und durch Wasserstoff ersetzt, und wenn erwünscht die anschliessend an das erste Verfahren genannten zusätzlichen Verfahrensschritte durchführt.

Ammonolytisch abspaltbare Substituenten und Reste $X_8$ sind Acylreste von organischen Carbonsäuren, z.B. Aroyl, wie Benzoyl, oder Niederalkanoyl, wie Acetyl.

Reaktionsfähige Derivate der in Formel VI definierten Carbonsäuren sind z.B. die Halogenide, wie die Chloride oder Bromide, ferner die Azide, sowie Säureanhydride insbesondere gemischte Säureanhydride mit z.B. Niederalkancarbonsäuren, wie Essigsäure oder Propionsäure, Niederalkoxyalkancarbonsäuren, wie 2-Methoxyessigsäure. Reaktionsfähige Derivate von Carbonsäuren der Formel VI sind insbesondere Ester, z.B. mit Niederalkanolen, wie Methanol, Aethanol, Isopropanol, tert.-Butanol, ferner mit Arylniederalkanolen, etwa gegebenenfalls durch Niederalkyl, z.B. Methyl, oder Niederalkoxy z.B. Methoxy, substituierter Benzylalkohol, oder Phenolen, die gegebenenfalls durch geeignete Substituenten aktiviert sind, z.B.

durch Halogen, etwa 4-Halogen, wie 4-Chlor, Niederalkoxy, etwa 4-Niederalkoxy wie 4-Methoxy, 4-Nitro oder 2,4-Dinitro, wie etwa 4-Chlorphenol, 4-Methoxyphenol, 4-Nitro- oder 2,4-Dinitrophenol, ferner mit Cycloalkanolen, wie etwa Cyclopentanol oder Cyclohexanol, die gegebenenfalls durch Niederalkyl, z.B. Methyl, substituiert sein können. Die Umsetzung wird in an sich bekannter Weise, üblicherweise in Gegenwart eines inerten Lösungsmittels z.B. in einem Temperaturbereich von etwa -10° bis +50°C in einem geschlossenen Gefäss durchgeführt.

Die Ausgangsstoffe der Formel VI lassen sich in an sich bekannter Weise erhalten, indem man eine Verbindung der Formel (II), in der $X_1$ und $Z_1$ zusammen die Epoxygruppe bedeuten, mit einer Aminoverbindung der Formel

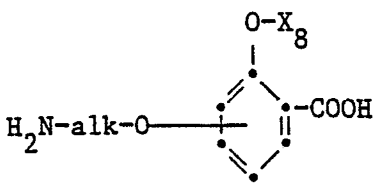

(VIa)

worin $X_8$ die angegebene Bedeutung hat, oder einem reaktionsfähigen Derivat davon, umsetzt.

Man kann ferner die durch Umsetzung einer Verbindung der Formel

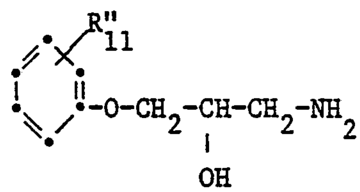

(VIb)

mit einer Carbonylverbindung der Formel

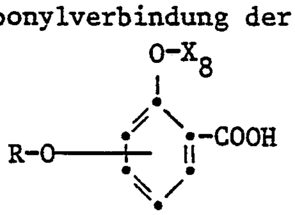

(VIc) ,

worin R den einem Alkylenrest Alk entsprechenden, an der Stelle von dessen freier Valenz einen Oxorest enthaltenden Alkylrest bedeutet, gebildete Schiff'sche Base mit einem Borhydrid, etwa Natriumborhydrid reduzieren. Die Reduktion kann auch mittels aktiviertem Wasserstoff in Gegenwart eines Hydrierkatalysators, z.B. eines Platin-auf-Kohle-Katalysators erfolgen.

Carbonylverbindungen der Formel (VIc) wiederum können durch Umsetzung einer Verbindung der Formel

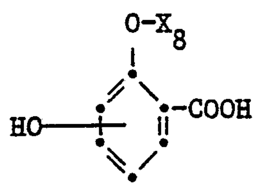

(Vld)

mit einer Verbindung der oben erläuterten Formel

R-Hal (IVg),

worin Hal für Halogen, insbesondere Chlor steht, in an sich bekannter Weise erhalten werden.

Die neuen Verbindungen der Formel I können ebenfalls erhalten werden, indem man in einer Verbindung der Formel

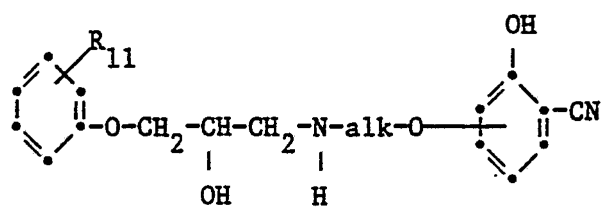

(VII),

worin eine oder alle Hydroxygruppen und/oder das sekundäre Stickstoffatom und/oder in 4-Stellung gebundenes Niederalkylsulfonylamino $R_{11}$ am Stickstoffatom gegebenenfalls durch solche mittels Hydrolyse abspaltbaren und durch Wasserstoff ersetzbaren Gruppen substituiert wird, die unter den Bedingungen des Verfahrens abgespalten und durch Wasserstoff ersetzt werden, die Gruppe -CN mittels Hydrolyse in die Gruppe $-CONH_2$ umwandelt und gleichzeitig gegebenenfalls am Stickstoffatom einer

in 4-Stellung gebundenen Niederalkylsulfonylaminogruppe und/oder an einer oder allen Hydroxygruppen und/oder am sekundären Stickstoffatom stehende Schutzgruppen abspaltet und durch Wasserstoff ersetzt, und, wenn erwünscht, die anschliessend an das erste Verfahren genannten zusätzlichen Verfahrensschritte durchführt.

Die obigen Reaktionen werden in an sich bekannter Weise durchgeführt. Die Hydrolyse wird in einem basischen oder vorteilhafterweise in einem sauren Medium, insbesondere in Gegenwart konzentrierter wässriger Mineralsäure, wie z.B. konz. Salzsäure, und wenn notwendig, unter Kühlen oder Erwärmen, z.B. in einem Temperaturbereich von etwa 0 bis 60°, vorzugsweise von etwa 40-50°, in einem offenen oder geschlossenen Gefäss und/oder in einer Inertgasatmosphäre, z.B. in einer Stickstoffatmosphäre durchgeführt.

Die Ausgangsstoffe der Formel VII lassen sich z.B. durch Umsetzen einer Verbindung der Formel

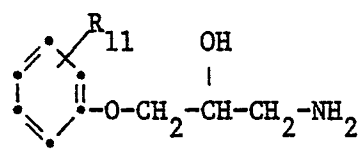

(VIIa)

mit einer Verbindung der Formel

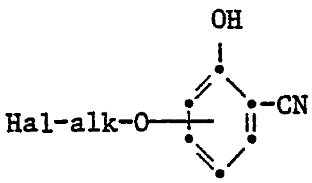

(VIIb),

worin Hal Chlor, Brom oder Jod darstellt, erhalten. Die Umsetzung wird vorteilhafterweise in Gegenwart eines basischen Mittels in an sich bekannter Weise durchgeführt.

Die Verbindung VIIb wiederum kann durch Einwirkung von Essigsäureanhydrid auf das dem Cyanid entsprechende Oxim erhalten werden. Dies geschieht zweckmässigerweise durch Kochen unter Rückfluss. Das Oxim kann seinerseits aus dem entsprechenden Aldehyd durch Kochen mit

Hydroxylamin-hydrochlorid in Gegenwart von alkoholischer Sodalösung unter Rückfluss hergestellt werden. Der entsprechende Aldehyd wiederum kann durch Umsetzung von 2,4-Dihydroxybenzaldehyd mit einem $\alpha$, $\omega$-Dihalogenniederalkan, vorzugsweise in Gegenwart eines basischen Mittels hergestellt werden. Man kann aber auch in analoger Weise ein Hydroxysalicylonitril, z.B. das 2,4-Dihydroxybenzonitril [Chem.Ber. 24, 3657 (1891)] oder das 2,5-Dihydroxybenzonitril [Helv. Chim.Acta 30, 149, 153 (1947)] mit einem nicht-geminalen Dihalogenniederalkan zu einer Verbindung der Formel VIIb umsetzen.

Die neuen Verbindungen der Formel I, worin $R_{11}$ Polyhydroxyniederalkyl, z.B. 1,2-Dihydroxy- oder 2,3-Dihydroxyniederalkyl, wie etwa 1,2-Dihydroxy- oder 2,3-Dihydroxypropyl darstellt, können erhalten werden, indem man in einer Verbindung der Formel

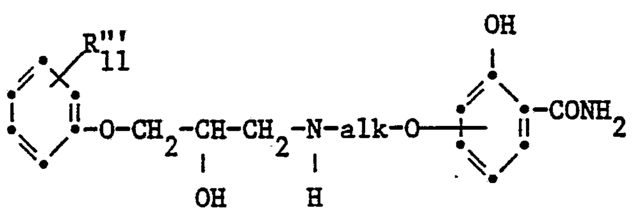

(VIII)

worin $R_{11}'''$ eine Polyhydroxyniederalkylgruppe, z.B. eine der genannten, bedeutet, worin mindestens eine, oder zwei Hydroxygruppen zusammen, wovon je eine an zwei benachbarten Kohlenstoffatomen steht, durch einen abspaltbaren und durch Wasserstoff ersetzbaren Rest geschützt ist, oder in einem Salz davon, diese Schutzgruppen, die gleich oder verschieden sein können, sowie gegebenenfalls weitere am Stickstoffatom und/oder an den Sauerstoffatomen stehende Schutzgruppen abspaltet und durch Wasserstoff ersetzt, und, wenn erwünscht, die anschliessend an das erste Verfahren genannten zusätzlichen Verfahrensschritte durchführt. Abspaltbare und durch Wasserstoff ersetzbare, z.B. mittels Solvolyse wie Hydrolyse, Alkoholyse oder Acidolyse, oder mittels Reduktion einschliesslich Hydrogenolyse, etwa wie oben beschrieben, abspaltbare Gruppen, sind z.B. die oben beschriebenen

Reste $X_2$ oder $X_4$. Solvolytisch wie hydrolytisch oder acidolytisch abspaltbare Reste $X_2$ oder $X_4$ sind beispielsweise Acylreste, wie Acylreste organischer Carbonsäuren, z.B. Niederalkanoyl wie Acetyl, oder Aroyl wie Benzoyl, ferner z.B. Niederalkoxycarbonyl, gegebenenfalls substituiertes 1-Phenylniederalkoxycarbonyl z.B. Benzyloxycarbonyl, ferner eine gegebenenfalls substituierte 1-Polyphenylniederalkylgruppe, z.B. Trityl, und ausserdem der Tetrahydropyranylrest. An zwei benachbarten Hydroxygruppen zusammen stehende Schutzgruppen sind z.B. Niederalkyliden, z.B. Methylen oder Isopropyliden, oder 1-Phenyl-niederalkyliden, dessen Phenylteil gegebenenfalls durch Niederalkyl, wie Methyl, oder Niederalkoxy, wie Methoxy, substituiert ist, insbesondere Benzyliden, oder Cycloalkyliden, z.B. Cyclopentyliden oder Cyclohexyliden, ferner die Carbonylgruppe.

Hydrolytisch abspaltbare Gruppen der angegebenen Art, z.B. Acylreste von organischen Carbonsäuren, z.B. Niederalkanoylreste, ferner z.B. Niederalkoxycarbonyl oder Tritylreste, ferner Tetrahydropyranylreste, ferner an zwei Hydroxygruppen zusammen gebundene Niederalkyliden-, 1-Phenyl-niederalkyliden- oder Cycloalkylidengruppen sowie gegebenenfalls weitere am Stickstoffatom und/oder an den Sauerstoffatomen stehende Schutzgruppen dieser Art können je nach Art solcher Reste durch Behandeln mit Wasser unter sauren oder basischen Bedingungen, z.B. in Gegenwart einer Mineralsäure, wie Chlorwasserstoff- oder Schwefelsäure, oder eines Alkalimetall- oder Erdalkalimetallhydroxids oder -carbonats abgespalten werden. Eine an zwei benachbarten Hydroxygruppen zusammen stehende Carbonylgruppe wird zweckmässigerweise mittels basischer Mittel, etwa eines Alkalihydroxids, wie z.B. Kaliumhydroxid, oder eines Alkalimetallalkoholats, wie Natriumäthylat oder Kalium-tert.-butylat, abgespalten, während z.B. Tetrahydropyranylreste mittels saurer Mittel, etwa wie angegeben, abgespalten werden. Acidolytisch abspaltbare Reste sind z.B. die oben für $X_2$ und/oder $X_4$ genannten und stellen beispielsweise Niederalkoxycarbonyl oder auch tert.-Niederalkylreste dar. Solche Reste können, z.B. wie oben beschrieben, durch Behandeln mit geeigneten starken organischen Carbonsäuren, wie gegebenenfalls durch Halogen, insbesondere Fluor, substituierten Niederalkancarbonsäuren, in erster Linie mit Trifluoressigsäure (wenn notwendig, in Gegenwart eines aktivierenden Mittels, wie Anisol), sowie mit Ameisensäure abgespalten werden. Diese Umsetzungen werden in an sich bekannter Weise vorgenommen.

Eine besonders geeignete hydrogenolytisch abspaltbare Hydroxyschutzgruppe ist in erster Linie eine hydrogenolytisch abspaltbare α-Arylniederalkylgruppe, wie eine gegebenenfalls substituierte 1-Polyphenylniederalkyl- oder 1-Phenylniederalkylgruppe, worin Substituenten, insbesondere des Phenylteils, z.B. Niederalkyl, wie Methyl, oder Niederalkoxy wie Methoxy sein können, und in erster Linie Benzyl. Eine an zwei benachbarten Hydroxygruppen zusammen stehende hydrogenolytisch abspaltbare Gruppe ist z.B. gegebenenfalls substituiertes 1-Phenylniederalkyliden, worin Substituenten insbesondere des Phenylteils, z.B. Niederalkyl, wie Methyl, oder Niederalkoxy, wie Methoxy, sein können, und insbesondere Benzyliden. Hydrogenolytisch abspaltbare Gruppen der genannten Art können auf übliche Weise durch Behandeln mit katalytisch aktiviertem Wasserstoff, z.B. mit Wasserstoff in Gegenwart eines Nickelkatalysators, wie Raney-Nickel, oder eines geeigneten Edelmetallkatalysators abgespalten werden.

An einer oder beiden Hydroxygruppen stehende, mittels Reduktion abspaltbare Schutzgruppen sind z.B. solche, die beim Behandeln mit einem chemischen Reduktionsmittel, z.B. wie oben beschrieben, abgespalten werden, z.B. 2-Halogenniederalkoxycarbonyl oder Arylmethoxycarbonyl.

Die Abspaltung erfolgt, etwa mittels der oben beschriebenen Methoden, z.B. mittels Zink, oder einem Chrom(II)salz, oder einer organischen Carbonsäure, wie Ameisensäure.

Weitere, am Stickstoffatom und/oder den Sauerstoffatomen gegebenenfalls vorhandene Schutzgruppen entsprechen den vorhin genannten und

mittels der beschriebenen Methoden abspaltbaren und durch Wasserstoff ersetzbaren Gruppen, wobei solche Gruppen im Zuge des beschriebenen Verfahrens gleichzeitig mit anderen Gruppen oder nachfolgend in einer getrennten Verfahrensmassnahme abgespalten werden.

Ausgangsstoffe der Formel VIII, worin $R_{11}'''$ einen in eine Polyhydroxyniederalkyl-, wie 1,2- oder 2,3-Dihydroxyniederalkylgruppe, z.B. eine 2,3-Dihydroxypropylgruppe, überführbaren Rest darstellt, können z.B. erhalten werden, indem man eine gegebenenfalls am Stickstoffatom und/ oder den Sauerstoffatomen Schutzgruppen tragende Verbindung der Formel VIII, worin $R_{11}'''$ z.B. einer Gruppe der Formel

$$-R_{10}-\underset{\displaystyle Z_4}{\underset{|}{CH}}-CH_2-Z_3 \qquad \text{(VIIIa)}$$

entspricht, in welcher $R_{10}$ einem zwei Kohlenstoffatome weniger aufweisenden Niederalkylrest entspricht, und mindestens eine der Gruppen $Z_3$ und $Z_4$, die gleich oder verschieden sein können, eine reaktionsfähige veresterte Hydroxygruppe, z.B. Halogen, wie Chlor und insbesondere Brom, darstellt, und die andere Hydroxy bedeutet, mit einem Salz einer Carbonsäure, z.B. einem Alkalimetallsalz, etwa dem Kaliumsalz, einer Niederalkancarbonsäure, z.B. Essigsäure, oder einer aromatischen Carbonsäure, wie Benzoesäure, zu der entsprechenden, eine oder zwei Acyloxygruppen in der Gruppe VIIIa enthaltenden Verbindung umsetzt. Ausgangsstoffe der Formel VIII mit einer Gruppe VIIIa wiederum können durch Addition von Halogen, etwa Brom, oder einer Halogenverbindung der Formel Hal-OH, etwa unterchloriger oder unterbromiger Säure, an einen der Gruppe VIIIa entsprechenden Alkenylrest erhalten werden. Diese Umsetzungen werden in üblicher Weise vorgenommen.

Ausgangsstoffe der Formel VIII, worin $R'''_{11}$ z.B. eine Gruppe der Formel

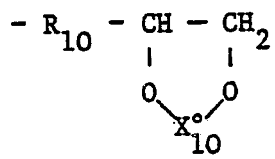 (VIIIb)

darstellt, worin $X^{\circ}_{10}$ einen hydrolytisch, einschliesslich alkoholytisch oder acidolytisch, oder mittels Reduktion einschliesslich Hydrogenolyse abspaltbaren Rest, z.B. einen der oben genannten darstellt, und $R_{10}$ die angegebene Bedeutung hat, können erhalten werden, indem man z.B. eine Verbindung der Formel

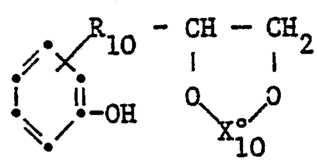 (VIIIc)

mit Epichlorhydrin umsetzt, und die erhaltene Verbindung der Formel

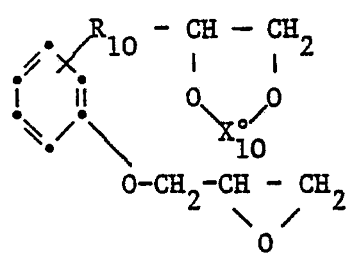 (VIIId)

z.B. mit einem gegebenenfalls N-geschützten, wie N-benzylierten 5-(2-aminoäthoxy)salicylamid zu einer Verbindung der Formel VIII umsetzt, worin $R'''_{11}$ die Gruppe VIIIb bedeutet.

Diese Umsetzungen werden in üblicher Weise, gegebenenfalls unter Kühlen oder Erwärmen und in einem geeigneten Lösungsmittel vorgenommen.

Die neuen Verbindungen der Formel I, worin $R_{11}$ eine 4-Niederalkylsulfonylaminogruppe darstellt, können ebenfalls erhalten werden, indem man in eine Verbindung der Formel

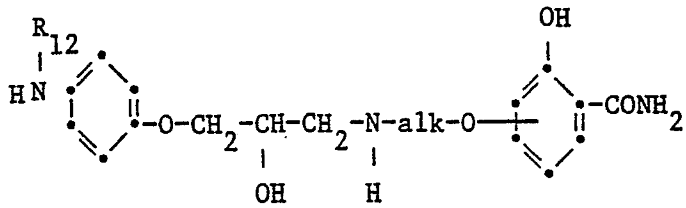 (IX) ,

worin $R_{12}$ Wasserstoff oder Niederalkyl bedeutet, die Niederalkylsulfonylgruppe einführt, und, wenn erwünscht, die anschliessend an das erste Verfahren beschriebenen zusätzlichen Verfahrensschritte durchführt. Die Einführung einer Niederalkylsulfonylgruppe erfolgt durch Umsetzung mit einer entsprechenden Niederalkansulfonsäure, z.B. Methansulfonsäure, gegebenenfalls in Gegenwart eines geeigneten Kondensationsmittels, z.B. N,N'-Dicyclohexylcarbodiimid. Anstelle einer Niederalkansulfonsäure, kann man auch ein reaktionfähiges Derivat davon, z.B. ein Halogenid, wie das Chlorid oder Bromid, ferner ein Azid, oder ein Säureanhydrid z.B. ein solches mit einer Niederalkancarbonsäure, wie Essigsäure, verwenden. Reaktionsfähige Derivate von Niederalkansulfonsäuren sind insbesondere auch Ester, z.B. solche mit Niederalkanolen wie Methanol, Aethanol, Isopropanol, tert.-Butanol, ferner mit Arylniederalkanolen, etwa gegebenenfalls durch Niederalkyl, z.B. Methyl, oder Niederalkoxy, z.B. Methoxy, substituiertem Benzylalkohol, oder mit Phenolen, die gegebenenfalls durch geeignete Substituenten, wie 4-Halogen, etwa 4-Chlor, 4-Nitro oder 4-Methoxy, aktiviert sind und demnach etwa 4-Methoxy-, 4-Nitro-, 2,4-Dinitro-, 4-Chlorphenol darstellen.

Zweckmässigerweise liegt die in der Seitenkette stehende sekundäre Aminogruppe in vermindert reaktionsfähiger, z.B. protonierter, Form vor, indem man die Umsetzung in einem pH-Bereich von etwa 4,0-5,0, beispielsweise in Gegenwart einer Säure, wie Essigsäure, vornimmt.

Diese Umsetzungen werden in an sich bekannter Weise, z.B. bei erhöhter oder erniedrigter Temperatur in Ab- oder Anwesenheit eines geeigneten Lösungsmittels, gegebenenfalls in einer Schutzgasatmosphäre, etwa unter Stickstoff, vorgenommen.

Ausgangsstoffe der Formel IX sind in üblicher Weise zugänglich, beispielsweise durch Umsetzung der Verbindung der Formel

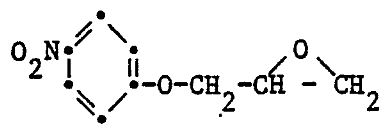

(IXa)

mit einer Verbindung der Formel

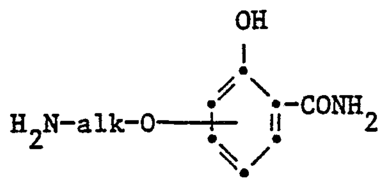

(IXb),

worin die primäre Aminogruppe und/oder die Hydroxygruppe durch eine hydrogenolytisch abspaltbare, durch Wasserstoff ersetzbare Gruppe, etwa, Benzyl, geschützt sein kann. Durch anschliessende Reduktion der Nitrogruppe zur Aminogruppe und gleichzeitiger oder in einem gesonderten Reduktionsschritt erfolgender Abspaltung einer in der erhaltenen Verbindung vorhandenen hydrogenolytisch abspaltbaren N- und/ oder O-Schutzgruppe wird das Ausgangsmaterial der Formel IX erhalten. Diese Reduktionen werden in üblicher Weise, z.B. mittels aktiviertem Wasserstoff, z.B. Wasserstoff in Gegenwart eines Katalysators, wie Nickel, oder eines Edelmetallkatalysators, wie Platin oder Palladium, gegebenenfalls auf einem geeigneten Trägermaterial, wie Kohle, durchgeführt.

Ausgangsstoffe der Formel IX, worin $R_{12}$ Niederalkyl bedeutet, können durch Umsetzung einer Verbindung der Formel

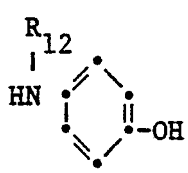

(IXc)

mit einer Niederalkansulfonsäure, oder einem reaktionsfähigen Derivat, etwa dem Chlorid, davon, Umsetzung der erhaltenen Verbindung der Formel

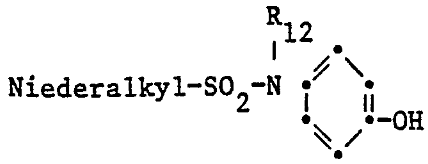 (IXd),

worin $R_{12}$ Niederalkyl darstellt, mit Epichlorhydrin zur entsprechenden Verbindung der Formel

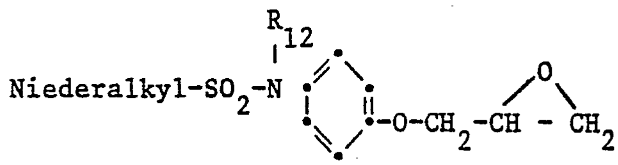 (IXe)

und deren Umsetzung mit einer Verbindung der Formel IXb erhalten werden.

Diese Reaktionen werden in an sich bekannter Weise durchgeführt.

Die neuen Verbindungen der Formel I können ebenfalls erhalten werden, indem man eine Verbindung der Formel

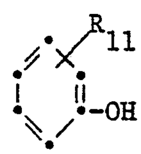 (X),

mit einer Verbindung der Formel

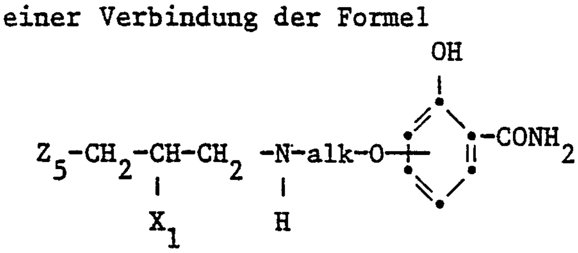 (XI),

worin $Z_5$ für eine reaktionsfähige, veresterte Hydroxygruppe steht und $X_1$ Hydroxy ist, oder worin $Z_5$ und $X_1$ zusammen die Epoxygruppe bedeuten, oder mit einer entsprechenden ringgeschlossenen Verbindung der Formel

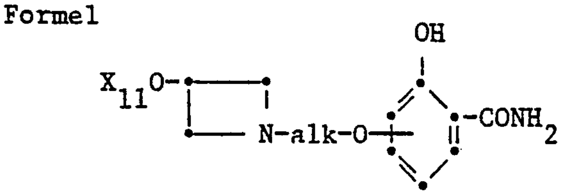 (XII),

worin $R_{11}$ und alk jeweils die angegebene Bedeutung haben, und $X_{11}$ für Wasserstoff oder eine unter den Bedingungen des Verfahrens abspaltbare und mit der Hydroxygruppe einer Verbindung der Formel X zur Bildung der Aetherbindung befähigte Gruppe steht, umsetzt, und, wenn erwünscht, die anschliessend an das erste Verfahren beschriebenen Verfahrensschritte durchführt. Im genannten Sinne abspaltbare und zur Bildung der Aetherbindung befähigte Gruppen $X_{11}$ sind z.B. Arylniederalkylgruppen, worin Aryl z.B. für Naphthyl, etwa 1-Naphthyl, und insbesondere für Phenyl steht, welches gegebenenfalls durch Niederalkyl, Niederalkoxy, Hydroxy, Halogen, Trifluormethyl und/oder Nitro substituiert ist. Im vorstehenden Sinne definierte Gruppen $X_{11}$ sind demnach insbesondere Benzyl, ferner Niederalkoxymethyl, z.B. Methoxymethyl.

Eine reaktionsfähige veresterte Hydroxygruppe $Z_5$ ist eine, durch eine starke Säure, insbesondere eine starke anorganische Säure, wie eine Halogenwasserstoffsäure, insbesondere Chlor-, Brom- oder Jodwasserstoffsäure, oder Schwefelsäure, oder eine starke organische Säure, insbesondere eine starke organische Sulfonsäure, wie eine aliphatische oder aromatische Sulfonsäure, z.B. Methansulfonsäure, 4-Methylphenylsulfonsäure oder 4-Bromphenylsulfonsäure, veresterte Hydroxygruppe, und stellt in erster Linie Halogen, z.B. Chlor, Brom oder Jod, oder aliphatisch oder aromatisch substituiertes Sulfonyloxy, z.B. Methylsulfonyloxy oder 4-Methylphenylsulfonyloxy dar.

Die Umsetzung einer Verbindung der Formel X mit einer solchen Formel XI wird in an sich bekannter Weise durchgeführt, wobei man, besonders bei Verwendung eines Ausgangsmaterials der Formel XI mit einer reaktionsfähigen veresterten Hydroxygruppe, vorteilhafterweise in Gegenwart eines basischen Mittels, wie einer anorganischen Base, z.B. eines Alkalimetall- oder Erdalkalimetallcarbonats oder -hydroxids, oder eines organischen basischen Mittels, wie eines Alkalimetallniederalkanolats, und/oder eines Ueberschusses des basischen Reaktionsteilnehmers arbeitet. Die Verbindung der Formel X

kann auch als Salz, etwa als Alkalisalz, z.B. als Natriumsalz vorliegen.

Die Umsetzung einer Verbindung der Formel X mit einer Verbindung der Formel XII erfolgt üblicherweise in Gegenwart eines basischen Katalysators, wie eines Alkalihydroxids, etwa Kaliumhydroxid, oder einer organischen Base, etwa Triäthylamin, oder eines sauren Katalysators, wie Trifluoressigsäure.

Diese Reaktionen werden in an sich bekannter Weise und üblicherweise in Gegenwart eines Lösungsmittels oder Lösungsmittelgemisches und, wenn notwendig, unter Kühlen oder Erwärmen, z.B. in einem Temperaturbereich von etwa +20° bis etwa +200°, vorzugsweise von etwa +100 bis etwa +180° in einem offenen oder geschlossenen Gefäss und/oder in einer Inertgasatmosphäre, z.B. in einer Stickstoffatmosphäre, durchgeführt.

Ausgangsstoffe der Formel XI, worin $Z_5$ eine reaktionsfähige veresterte Hydroxygruppe, z.B. Chlor ist, und $X_1$ Hydroxy darstellt, sind durch Umsetzung eines Amins mit gegebenenfalls geschützter Hydroxygruppe der Formel

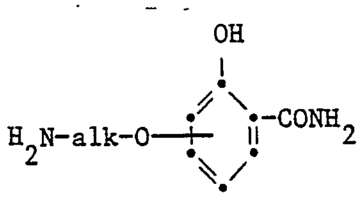

(XIa)

mit Epichlorhydrin in üblicher Weise zugänglich, während ein Ausgangsmaterial der Formel XI, worin $Z_5$ und $X_1$ zusammen die Epoxygruppe darstellen, aus einem Amin der Formel XIa mit Epichlorhydrin in Gegenwart eines basischen Mittels, z.B. eines Alkali- oder Erdalkalihydroxids oder -carbonats, wie Natriumhydroxid oder -carbonat, oder Calciumhydroxid oder -carbonat erhalten werden können. Eine geschützte Hydroxygruppe ist z.B. eine durch eine mittels Reduktion einschliesslich Hydrogenolyse, z.B. eine α-Arylniederalkylgruppe, wie eine gegebenenfalls, etwa wie oben angegeben, substituierte 1-Phenylniederalkylgruppe, z.B. eine Benzylgruppe, oder eine mittels Hydrolyse abspaltbare und durch Wasserstoff ersetzbare Gruppe, z.B. ein Acylrest, etwa

ein Niederalkanoylrest, wie eine Acetylgruppe, substituierte Hydroxygruppe. Solche Schutzgruppen werden mittels Reduktion, einschliesslich Hydrogenolyse, oder Hydrolyse, in üblicher Weise gleichzeitig oder in einem gesonderten Reaktionsschritt, z.B. wie beschrieben, abgespalten und durch Wasserstoff ersetzt.

Ausgangsstoffe der Formel XII können auf an sich bekannter Weise durch Umsetzung einer Verbindung der Formel

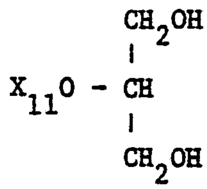

(XIIa),

worin die beiden Hydroxygruppen vorzugsweise in reaktionsfähiger, veresterter Form, z.B. als Halogen, wie Chlor, oder als Sulfonyloxywie Methansulfonyloxygruppe vorliegen können, und $X_{11}$ vorzugsweise verschieden von Wasserstoff ist und z.B., wie beschrieben, eine gegebenenfalls substituierte Benzylgruppe darstellen kann, mit einer Verbindung der Formel

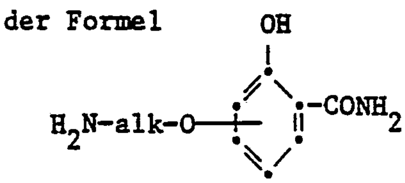

(XIIb),

worin alk die angegebene Bedeutung hat, erhalten werden. Diese Umsetzungen werden üblicherweise in einem Lösungsmittel oder Lösungsmittelgemisch, und, wenn notwendig, unter Kühlen oder Erwärmen, z.B. in einem Temperaturbereich von etwa -20° bis etwa +150°C, vorzugsweise von etwa +60° bis etwa +100°C, in einem offenen oder geschlossenen Gefäss und/oder in einer Inertgasatmosphäre, z.B. in einer Stickstoffatmosphäre, durchgeführt. Stellt in einem so erhaltenen Ausgangsmaterial der Formel XII die Gruppe $X_{11}$ eine gegebenenfalls, z.B. wie beschrieben, substituierte Benzylgruppe dar, so kann diese z.B. mittels katalytisch aktiviertem Wasserstoff, etwa Wasserstoff in Gegenwart eines Edelmetallkatalysators, wie eines Palladium-auf-Kohle-Katalysators, auf übliche Weise abgespalten und durch Wasserstoff ersetzt werden, wobei ein Ausgangsstoff der Formel XII, worin $X_{11}$ Wasserstoff ist, erhalten wird.

Die oben beschriebenen Reaktionen können gegebenenfalls gleichzeitig oder nacheinander, ferner in beliebiger Reihenfolge durchgeführt werden. Falls notwendig, erfolgen sie in Anwesenheit von Verdünnungsmitteln, Kondensationsmitteln und/oder katalytisch wirkenden Mitteln, bei erniedrigter oder erhöhter Temperatur, im geschlossenen Gefäss unter Druck und/oder in einer Inertgasatmosphäre.

Je nach den Verfahrensbedingungen und Ausgangsstoffen erhält man die neuen Verbindungen in freier Form oder in der ebenfalls von der Erfindung umfassten Form ihrer Salze, wobei die neuen Verbindungen oder Salze davon auch als Hemi-, Mono-, Sesqui- oder Polyhydrate davon vorliegen können. Säureadditionssalze der neuen Verbindungen können in an sich bekannter Weise, z.B. durch Behandeln mit basischen Mitteln, wie Alkalimetallhydroxiden, -carbonaten oder -hydrogencarbonaten oder Ionenaustauschern, in die freien Verbindungen übergeführt werden. Andererseits können erhaltene freie Basen mit organischen oder anorganischen Säuren, z.B. mit den genannten Säuren, Säureadditionssalze bilden, wobei zu deren Herstellung insbesondere solche Säuren verwendet werden, die sich zur Bildung von pharmazeutisch annehmbaren Salzen eignen.

Diese oder andere Salze, insbesondere Säureadditionssalze der neuen Verbindungen, wie z.B. Oxalate oder Perchlorate, können auch zur Reinigung der erhaltenen freien Basen dienen, indem man die freien Basen in Salze überführt, diese abtrennt und reinigt, und aus den Salzen wiederum die Basen freisetzt.

Die neuen Verbindungen können je nach der Wahl der Ausgangsstoffe und Arbeitsweisen, als optische Antipoden oder Racemate, oder sofern sie mindestens zwei asymmetrische Kohlenstoffatome enthalten, auch als Racematgemische vorliegen. Die Ausgangsstoffe können auch als bestimmte optische Antipoden eingesetzt werden.

Erhaltene Racematgemische können auf Grund der physikalisch-chemischen Unterschiede der Diastereoisomeren in bekannter Weise, z.B. durch Chromatographie und/oder fraktionierte Kristallisation, in die beiden stereoisomeren (diastereomeren) Racemate aufgetrennt werden.

Erhaltene Racemate lassen sich nach an sich bekannten Methoden in die Antipoden zerlegen, z.B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säuren, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Säuren sind z.B. die D- und L-Formen von Weinsäure, Di-O,O-(p-Toluoyl)-weinsäure, Aepfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man einen Ausgangsstoff unter den Reaktionsbedingungen bildet, oder bei denen eine Reaktionskomponente gegebenenfalls in Form ihrer Salze vorliegt.

Zweckmässig verwendet man für die Durchführung der erfindungsgemässen Reaktionen solche Ausgangsstoffe, die zu den eingangs besonders erwähnten Gruppen von Endstoffen und besonders zu den speziell beschriebenen oder hervorgehobenen Endstoffen führen.

Die Ausgangsstoffe sind bekannt oder können falls sie neu sind, nach an sich bekannten Methoden, wie oben, z.B. analog wie in den Beispielen beschrieben, erhalten werden. Die Ausgangsstoffe der Formeln II-IX und XI-XII, die speziell für die Herstellung der erfindungsgemässen Verbindungen der Formel I entwickelt wurden, sind noch neu und bilden ebenfalls einen Gegenstand der Erfindung.

Die Erfindung betrifft auch verfahrensgemäss erhältliche Zwischenprodukte.

Die neuen Verbindungen können z.B. in Form pharmazeutischer Präparate Verwendung finden, welche eine pharmakologisch wirksame Menge der Aktivsubstanz, gegebenenfalls zusammen mit pharmazeutisch verwendbaren Trägerstoffen enthalten, die sich zur enteralen, z.B. oralen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycerin und/oder Schmiermitteln, z.B. Kieselerde, Talk, Stearinsäure oder Salze davon, wie Magnesium oder Calciumstearat, und/oder Polyäthylenglykol, aufweisen. Tabletten können ebenfalls Bindemittel, z.B. Magnesiumaluminiumsilikat, Stärken, wie Mais-, Weizen-, Reis- und Pfeilwurzelstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die neuen pharmakologisch wirksamen Verbindungen in Form von parenteral verabreichbaren Präparaten oder von Infusionslösungen verwenden. Solche Lösungen sind vorzugsweise isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-,

Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wirksame Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes.

Die Dosierung kann von verschiedenen Faktoren, wie Applikationsweise, Spezies, Alter und/oder individuellen Zustand abhängen. So liegen die täglich in einer oder mehreren, vorzugsweise höchstens 4 Einzeldosen zu verabreichenden Dosen bei oraler Applikation an Warmblüter für β-Rezeptoren-Blocker der Formel I zwischen 0,03 und 3 mg/kg und für Warmblüter von etwa 70 kg vorzugsweise zwischen etwa 0,005 und etwa 0,3 g.

Die folgenden Beispiele dienen zur Illustration der Erfindung; Temperaturen werden in Celsiusgraden angegeben.

Beispiel 1: Eine Lösung von 6,9 g 1-[2-(3-Carbamoyl-4-hydroxy-phenoxy)-äthylamino]-3-[2-[(2,2-dimethyl-dioxolan-4-yl)-methyl]-phenoxy]-2-propanol in 165 ml 0,1-n.Salzsäure wird bei 20° 5 Stunden stehen gelassen, anschliessend 2-mal mit Aether gewaschen, die wässrige Phase filtriert und darauf im Vakuum bei einer Badtemperatur von 40° konzentriert. Die erhaltene wässerige Lösung wird lyophilisiert, wonach man 1-[2-(3-Carbamoyl-4-hydroxy-phenoxy)-äthylamino]-3-[2-(2,3-dihydroxy-propyl)-phenoxy]-2-propanol-hydrochlorid als farbloses amorphes Pulver erhält.

Das Ausgangsmaterial kann auf folgende Weise hergestellt werden:

1a) Ein Gemisch von 76,8 g 4-(2-Hydroxybenzyl)-2,2-dimethyl-1,3-dioxolan, 248 ml Epichlorhydrin und 69,7 g Kaliumcarbonat wird bei einer Badtemperatur von 130° während 7 Stunden gerührt.

Das Reaktionsgemisch wird filtriert und unter Vakuum zur Trockne eingeengt. Der Rückstand wird in Aether gelöst, die Lösung mit 2-n. Natronlauge, dann mit Wasser gewaschen, über Natriumsulfat getrocknet und anschliessend eingeengt. Das erhaltene rohe 4-[2-(2,3-Epoxipropoxy)-benzyl]-2,2-dimethyl-1,3-dioxolan wird als solches weiterverarbeitet.

1b) Ein Gemisch von 17,4 g der erhaltenen rohen Verbindung und 17,1 g 5-(2-Benzylamino-äthoxy)-salicylamid in 60 ml Dimethylsulfoxid wird bei einer Badtemperatur von 80° während 20 Stunden gerührt. Das Reaktionsgemisch wird auf Eis und Wasser gegossen und mit Essigsäureäthylester extrahiert. Die organische Phase wird mit Aether verdünnt und nacheinander mit 0,1-n.Salzsäure (bei pH 4-5), dann mit wässriger gesättigter Kaliumcarbonatlösung gewaschen und über Natriumsulfat getrocknet. Nach dem Eindampfen verbleibt ein Oel, das über Silicagel mit Methylenchlorid-Methanol chromatographiert wird. Nach dem Aufarbeiten erhält man 1-[N-[2-(3-Carbamoyl-4-hydroxy-phenoxy)-äthyl]-benzylamino]-3-[2[(2,2-dimethyl-dioxolan-4-yl)-methyl]-phenoxy]-2-propanol als dickes Oel.

1c) Eine Lösung von 23,5 g der erhaltenen Verbindung in 240 ml Methanol wird unter Zusatz von 2,4 g Palladium-auf-Kohle-Katalysator unter Normalbedingungen hydriert. Das Reaktionsprodukt wird in Essigsäureäthylester gelöst und durch Zugabe von Petroläther kristallin ausgefällt. Die Kristalle werden nochmals in Essigsäureäthylester gelöst und mit 4.5 g Kieselgel während 1/2 Stunde gerührt. Die filtrierte Lösung wird portionenweise mit Petroläther versetzt, wonach man 1-[2-(3-Carbamoyl-4-hydroxy-phenoxy)-äthylamino]-3-[2-[(2,2-dimethyl-dioxolan-4-yl)-methyl]-phenoxy]-2-propanol vom Smp. 91-93° erhält.

Beispiel 2: Eine Lösung von 20,0 g rohem 1-[N-[2-(4-Carbamoyl-3-hydroxy-phenoxy)-1-methyl-äthyl]-benzylamino]-3-(4-methansulfonylamino-phenoxy)-2-propanol in 250 ml Methanol wird nach Zusatz von 2,5 g Pd/C-Katalysator (5%) bei Normalbedingungen hydriert, bis die Debenzylierung beendet ist, (DC-Kontrolle), wozu noch ein Zusatz von weiteren 1,0 g des Katalysators benötigt wird.
Der Katalysator wird abfiltriert, das Filtrat eingedampft und das verbleibende Oel in wenig heissem Isopropanol gelöst. Nach dem Abkühlen erhält man kristallines 1-[2-(4-Carbamoyl-3-hydroxy-phenoxy)-1-methyl-äthylamino]-3-(4-methansulfonylamino-phenoxy)-2-propanol vom Smp. 147-150° (Diastereomeren-Gemisch).

Der Ausgangsstoff wird auf folgende Weise erhalten:

2a) Nach der von Irvine et al., Synthesis 1972, 568 beschriebenen Methode wird 2,4-Dihydroxybenzamid unter Verwendung eines Ueberschusses an Aceton in das 2,3-Dihydro-2,2-dimethyl-7-hydroxy-4H-1,3-benzoxazin-4-on vom Smp. 249-251° umgewandelt.

2b) Aus 168 g 2,3-Dihydro-2,2-dimethyl-7-hydroxy-4H-1,3-benzoxazin-4-on, 305 g Kaliumcarbonat und 88 ml Chloraceton in 1,2 Liter Acetonitril erhält man durch Kochen während 28 Stunden und nachfolgendem Aufarbeiten das 2,3-Dihydro-2,2-dimethyl-7-(2-oxo-propoxy)-4H-1,3-benzoxazin-4-on vom Smp. 160-162° (aus Isopropanol).

2c) Eine Lösung von 75 g rohem 2,3-Dihydro-2,2-dimethyl-7-(2-oxo-propoxy)-4H-1,3-benzoxazin-4-on und 32 g Benzylamin in 1000 ml Methanol wird unter Zusatz von 0,75 g konz. Schwefelsäure und 1,6 g Pt/C-Katalysator (5%) bis zum Stillstand der Wasserstoffaufnahme unter Normalbedingungen hydriert. Nach Abfiltrieren des Katalysators und Abdampfen des Lösungsmittels wird der ölige Rückstand zwischen 300 ml Aethylacetat und 500 ml 2-n.Salzsäure verteilt. Aus der wässrigen Phase isoliert man durch versetzen mit konz. Ammoniak (Eiskühlung) und Extraktion mit Aethylacetat

rohes 2,3-Dihydro-2,2-dimethyl-7-[(2-benzylamino)-propoxy] -4H-1,3-benzoxazin-4-on als Oel, welches roh weiterverwendet werden kann.

2d) Ein Gemisch von 100 g rohem 2,3-Dihydro-2,2-dimethyl-7-[(2-benzylamino)-propoxy]-4H-1,3-benzoxazin-4-on, 100 ml Isopropanol und 100 ml Isopropylamin wird 1 Stunde am Rückfluss gekocht und dann eingedampft. Das verbleibende Oel kristallisiert beim Verreiben mit Aether. Die Kristalle werden abgenutscht und mit wenig Isopropanol gewaschen. Man erhält so 4-[2-(Benzylamino)-propoxy]-salicylamid vom Smp. 121-123°.

2e) Eine Lösung von 19,0 g 4-[2-(Benzylamino)-propoxy]-salicylamid und 12,4 g 4-(2,3-Epoxypropoxy)-nitrobenzol in 300 ml Isopropanol wird 20 Stunden unter Rückfluss gekocht. Nach Zusatz von weiteren 1,2 g 4-(2,3-Epoxypropoxy)-nitrobenzol wird während weiteren 20 Stunden unter Rückfluss erhitzt. Anschliessend wird das Lösungsmittel zum Teil abgedampft, worauf 1-[N-[2-(4-Carbamoyl-3-hydroxy-phenoxy)-1-methyl-äthyl]-benzylamino]-3-(4-nitrophenoxy)-2-propanol vom Smp. 160-164° (Diastereomeren-Gemisch) auskristallisiert.

2f) Eine Lösung von 19,0 g der erhaltenen Verbindung in 380 ml Dioxan wird unter Zusatz von 18 g Raney-Nickel in 4 Portionen bei Normalbedingungen bis zur Aufnahme von 3 Mol-Aequivalent Wasserstoff hydriert. Nach Filtration und Eindampfen des Filtrates verbleibt rohes 1-[N-[2-(4-Carbamoyl-3-hydroxy-phenoxy)-1-methyl-äthyl]-benzylamino]-3-(4-amino-phenoxy)-2-propanol als orange-braunes Oel, das ohne weitere Reinigung weiterverarbeitet wird.

2g) 16 g der erhaltenen Verbindung werden in 90 ml wasserfreiem Pyridin gelöst, und hierzu unter Kühlen auf 5-10° 4,4 g Methansulfonsäurechlorid zugetropft. Das Reaktionsgemisch wird 4 Stunden bei Raumtemperatur gerührt, das Lösungsmittel abgedampft

und der Rückstand zwischen 400 ml Aethylacetat und 50 ml Wasser verteilt. Die organische Phase wird noch dreimal mit je 50 ml Wasser gewaschen, über Magnesiumsulfat getrocknet, mit Kohle behandelt und eingedampft, wonach man rohes 1-[N-[2-(4-Carbamoyl-3-hydroxy-phenoxy)-1-methyl-äthyl]-benzylamino]-3-(4-methansulfonylamino-phenoxy)-2-propanol als oranges Oel, erhält, welches ohne weitere Reinigung weiterverarbeitet wird.

Beispiel 3: Eine Lösung von 30 g rohem 1-[N-[2-(3-Carbamoyl-4-hydroxy-phenoxy)-äthyl]-benzylamino]-3-[4-[2-(cyclopropylmethoxy)-äthyl]-phenoxy]-2-propanol in 600 ml Methanol wird unter Zusatz von 4 g Pd/C-Katalysator (5%) bis zur Aufnahme von 1-Mol-Aequivalent Wasserstoff unter Normalbedingungen hydriert. Durch Zusatz von Dioxan und Erwärmen wird das bereits auskristallisierte Produkt in Lösung gebracht, der Katalysator abfiltriert und das Filtrat eingedampft. Nach dem Umkristallisieren aus Isopropanol, dann aus Methanol, erhält man reines 1-[2-(3-Carbamoyl-4-hydroxy-phenoxy)-äthylamino]-3-[4-[2-(cyclopropylmethoxy)-äthyl]-phenoxy]-2-propanol vom Smp. 149-150°.

Der Ausgangsstoff wird auf folgende Weise hergestellt:

3a) Nach der von Irvine et al., Synthesis 1972, 568 beschriebenen Methode wird 2,5-Dihydroxy-benzamid unter Verwendung eines Ueberschusses von Aceton in 2,3-Dihydro-2,2-dimethyl-6-hydroxy-4H-1,3-benzoxazin-4-on vom Smp. 215-216° übergeführt.

3b) 70 g 2,3-Dihydro-2,2-dimethyl-6-hydroxy-4H-1,3-benzoxazin-4-on werden in 400 ml Acetonitril mit 100 g Kaliumcarbonat und 32 ml Chloraceton 30 Stunden unter Rückfluss gerührt. Nach Zusatz von weiteren 3,2 ml Chloraceton wird das Reaktionsgemisch während weiter weiteren 15-20 Stunden erhitzt. Das noch warme Reaktionsgemisch wird filtriert, der Rückstand mit Aceton gründlich gewaschen und

das vereinigte Filtrat eingedampft. Der kristalline Rückstand wird aus Toluol umkristallisiert und ergibt das 2,3-Dihydro-2,2-dimethyl-6-(2-oxopropoxy)-4H-1,3-benzoxazin-4-on vom Smp. 125-126°.

3c) 74 g des erhaltenen 2,3-Dihydro-2,2-dimethyl-6-(2-oxo-propoxy)-4H-1,3-benzoxazin-4-on werden in einem Gemisch von 150 ml Dioxan und 450 ml 2-n.Salzsäure 45 Minuten auf dem Wasserbad erhitzt. Das Lösungsmittel wird abgedampft und der kristalline Rückstand mit Wasser verrieben und dann abgesaugt. Durch Umkristallisation aus Isopropanol erhält man das 5-(2-Oxo-propoxy)-Salicylamid vom Smp. 152-154°.

3d) Eine Lösung von 104,5 g 5-(2-Oxo-propoxy)-salicylamid in 1000 ml Methanol wird mit 55 g Benzylamin und 1,25 g konz. Schwefelsäure versetzt und in Anwesenheit von 3,0 g Pt/C-Katalysator unter Normalbedingungen bis zur Aufnahme von 1 Aequivalent Wasserstoff hydriert. Der Katalysator wird abfiltriert, die Lösung mit ca. 10 g pulverisiertem Calciumcarbonat verrührt, nochmals filtriert und eingedampft. Das verbleibende Oel kristallisiert aus Isopropanol. Nochmalige Umkristallisation aus Isopropanol ergibt 5-[2-(Benzylamino)-propoxy]-salicylamid vom Smp. 102-104°.

3e) Eine Lösung von 14,3 g 1-[2-(Cyclopropyl-methoxy)-äthyl]-4-(2,3-epoxypropoxy)-benzol (DE-OS 26 49 605) und 16,3 g 5-[2-(Benzylamino)-propoxy]-salicylamid in 200 ml Isopropanol wird 18 Stunden unter Rückfluss gekocht. Nach dem Eindampfen des Reaktionsgemisches erhält man rohes 1-[N-[2-(3-Carbamoyl-4-hydroxy-phenoxy)-äthyl]-benzylamino]-3-[4-[2-(cyclopropylmethoxy)-äthyl]-phenoxy]-2-propanol als braunes Oel, das ohne weitere Reinigung weiterverarbeitet wird.

Beispiel 4: Eine Lösung von 50 g rohem 1-[N-[2-(3-Carbamoyl-4-hydroxy--phenoxy)-äthyl]-benzylamino]-3-(4-methansulfonylamino-phenoxy)-2-propanol in 550 ml Methanol wird nach Zusatz von 6 g Palladium-auf-Kohle-Katalysator (5%) bei Normalbedingungen hydriert, bis die Wasserstoffaufnahme zum Stillstand gekommen ist. Das bereits auskristallisierte Produkt wird durch Zusatz von Dioxan und Erwärmen in Lösung gebracht, der Katalysator abfiltriert und das Filtrat eingedampft. Der kristalline Eindampfrückstand ergibt nach dem Umkristallisieren aus Aethanol, dann aus Methanol unter Zusatz von Aktivkohle das 1-[2-(3-Carbamoyl-4-hydroxy-phenoxy)-äthylamino]-3-(4-methansulfonylamino-phenoxy)-2-propanol vom Smp. 152-153°.

Das Ausgangsmaterial wird auf folgende Weise hergestellt:

4a) 19,5 g 4-(2,3-Epoxypropoxy)-nitrobenzol und 28,6 g 5-(2-Benzyl-aminoäthoxy)-salicylamid werden in 400 ml Isopropanol 20 Stunden unter Rückfluss gekocht. Das nach dem Eindampfen des Reaktionsgemischs als zähes Oel erhaltene rohe 1-[N-[2-(3-Carbamoyl-4-hydroxy-phenoxy)-äthyl]-benzylamino]-3-(4-nitro-phenoxy)-2-propanol wird als solches weiterverarbeitet.

4b) 50 g der nach Beispiel 4a) erhaltenen rohen Verbindung werden in 500 ml Aethanol gelöst und in Gegenwart von 5 g Raney-Nickel hydriert. Es werden noch 2 Zusätze von je 10 g Raney-Nickel benötigt, bis die berechnete Menge (3 Mol-Aequiv.) Wasserstoff aufgenommen ist. Nach dem Abfiltrieren und Eindampfen des Filtrats erhält man rohes 1-[N-[2-(3-Carbamoyl-4-hydroxy-phenoxy)-äthyl]-benzyl-amino]-3-(4-amino-phenoxy)-2-propanol als braunes Oel.

4c) 44 g der nach Beispiel 4b) erhaltenen rohen Verbindung werden in 300 ml wasserfreiem Pyridin gelöst und unter Rühren und Kühlen bei 10-20° tropfenweise mit 11,4 g Methansulfonsäurechlorid versetzt. Das Reaktionsgemisch wird über Nacht bei Raumtemperatur gerührt, eingedampft und zwischen 500 ml Aethylacetat und 100 ml

Wasser verteilt. Die organische Phase wird abgetrennt, zweimal mit je 100 ml Wasser gewaschen, über Magnesiumsulfat getrocknet und eingedampft. Das so als zähes Oel erhaltene rohe 1-[N-[2-(3-Carbamoyl-4-hydroxy-phenoxy)-äthyl]-benzylamino]-3-(4-methansulfonylamino-phenoxy)-2-propanol wird ohne weitere Reinigung weiterverarbeitet.

Beispiel 5: Ein Gemisch von 11,5 g 4-(2,3-Epoxy-propoxy)-zimtsäure-N-methylamid und 7,4 g 5-(2-Aminoäthoxy)-salicylamid wird in 50 ml auf ca. 80° erwärmtem Dimethylsulfoxid gelöst, die Lösung 1 Stunde bei 75-85° gerührt und hierauf auf 500 ml Wasser gegossen. Das sich abscheidende harzartige Produkt wird abgetrennt und mit 150 ml Aethylacetat verrührt. Die sich abscheidenden Kristalle werden abgesaugt und aus wenig Isopropanol umkristallisiert, wonach man das 4-[3-[2-(3-Carbamoyl-4-hydroxy-phenoxy)-äthylamino]-2-hydroxy-propoxy]-N-methylzimtsäureamid vom Smp. 170-171° (sintert ab 148°) erhält.

Beispiel 6: Eine Lösung von 41 g rohem 1-[N-[2-(3-Carbamoyl-4-hydroxy-phenoxy)-äthyl]-benzylamino]-3-[4-(N-methyl-methansulfonylamino)-phenoxy]-2-propanol in 410 ml Methanol wird analog Beispiel 4 in Gegenwart von 4 g Palladium-auf-Kohle-Katalysator hydriert und aufgearbeitet. Man erhält nach dem Umkristallisieren aus Aethanol und Methanol das 1-[2-(3-Carbamoyl-4-hydroxy-phenoxy)-äthylamino]-3-[4-(N-methyl-methansulfonylamino)-phenoxy]-2-propanol vom Smp. 120-121°.

Das Ausgangsmaterial wird auf folgende Weise hergestellt:

6a) Eine Lösung von 103,7 g 4-Methylaminophenol-sulfat in 330 ml Pyridin und 102 ml N,N-Diisopropyläthylamin wird unter Eiskühlung und Rühren mit 77 ml Methansulfonsäurechlorid während ca. 30 Minuten tropfenweise versetzt und bei Raumtemperatur über Nacht gerührt. Die flüchtigen Anteile werden abgedampft, der Eindampfrückstand zwischen Aethylacetat und Wasser verteilt, die organische Phase abgetrennt und eingedampft und der kristalline Rückstand mit 300 ml 6-n. Natronlauge bis zur völligen Lösung auf dem Wasserbad erwärmt. Die Lösung wird filtriert und mit konz. Salzsäure auf pH 2 eingestellt. Hierbei fällt das 4-(N-Methylsulfonylamino)-phenol kristallin aus. Es wird abgesaugt und im Vakuum bei 80° getrocknet; Smp. 135-136°.

6b) Ein Gemisch von 34,2 g der nach Beispiel 6a) erhaltenen Verbindung, 35,2 g Kaliumcarbonat und 125 ml Epichlorhydrin wird 2 Stunden unter Rühren und Rückfluss gekocht. Die Suspension wird filtriert, das Filtrat eingedampft, zwischen Aethylacetat und Wasser verteilt, die organische Phase abgetrennt, über Magnesiumsulfat getrocknet und eingedampft. Man erhält das 4-(2,3-Epoxypropoxy)-N-methyl-methansulfonanilid vom Smp. 96-100° (aus Methanol).

6c) Eine Lösung von 19,0 g der nach Beispiel 6b) erhaltenen Verbindung und 21,4 g 5-(2-Benzylaminoäthoxy)-salicylamid in 350 ml Isopropanol wird 5 Stunden unter Rückfluss gekocht. Nach dem Eindampfen erhält man rohes 1-[N-[2-(3-Carbamoyl-4-hydroxy-phenoxy)-äthyl]-benzylamino]-3-[4-(N-methyl-methansulfonylamino)-phenoxy]-2-propanol als zähflüssiges Oel, welches als solches weiterverarbeitet wird.

Beispiel 7: Ein Gemisch von 5 g Methansulfonylchlorid, 5 g 1-[2-(3-Carbamoyl-4-hydroxy-phenoxy)-äthylamino]-3-(4-aminophenoxy)-2-propanol, 1,5 g Essigsäure und 100 ml Wasser wird auf dem siedenden Wasserbad unter Rühren während 15 Minuten erwärmt. Nach dem Erkalten stellt man durch Zufügen von konz. Natronlauge auf pH 9,0-12,0 ein und extrahiert erschöpfend mit Aethylacetat. Die Aethylacetatlösung wird mit Wasser gewaschen, über Natriumsulfat getrocknet und zur Trockne abgedampft. Der Eindampfrückstand wird aus Aethanol, dann aus Methanol umkristallisiert und ergibt das 1-[2-(3-Carbamoyl-4-hydroxy-phenoxy)-äthylamino]-3-(4-methansulfonylamino-phenoxy)-2-propanol vom Smp. 152°.

Das Ausgangsmaterial kann auf folgende Weise hergestellt werden:

7a) Eine Lösung von 10 g des nach Beispiel 4b) erhaltenen 1-[N-[2-(3-Carbamoyl-4-hydroxy-phenoxy)-äthyl]-benzylamino]-3-(4-amino-phenoxy)-2-propanol in 100 ml Aethanol wird nach Zugabe von 0,5 g Palladium-auf-Kohle-Katalysator (5%-ig) bis zur beendeten Wasserstoffaufnahme (1 Mol-Aequivalent) hydriert. Nach dem Abfiltrieren des Katalysators wird das Filtrat zur Trockne verdampft, wonach man das 1-[2-(3-Carbamoyl-4-hydroxy-phenoxy)-äthylamino]-3-(4-aminophenoxy)-2-propanol als Rohprodukt erhält, welches als solches weiterverarbeitet wird.

<u>Beispiel 8:</u> Ein Gemisch von 13,9 g 1-(4-Methansulfonylamino-phenoxy)-3-amino-2-propanol und 10,5 g 4-(2-Oxo-propoxy)-salicylamid wird in 200 ml Toluol unter Zusatz von einigen Tropfen Essigsäure am Wasserabscheider gekocht. Nach Aufhören der Wasserabspaltung (~2-3 Stunden) wird die Lösung eingedampft, der dunkelrote Rückstand in 300 ml Aethanol gelöst und portionenweise unter Rühren mit insgesamt 5,7 g Natriumborhydrid versetzt. Die Temperatur steigt hierbei bis 36°. Das Reaktionsgemisch wird noch 2 Stunden bei 20-30° weitergerührt und über Nacht stehen gelassen. Unter Eiskühlung wird es dann mit ca. 6-n. Salzsäure auf pH 3-4 gebracht, filtriert und eingedampft. Der Eindampfrückstand wird zwischen je 100 ml Wasser und Aethylacetat verteilt, die Wasserphase wird abgetrennt, mit konz. Ammoniak alkalisch gestellt und mit 200 ml Aethylacetat extrahiert. Nach dem Waschen der organischen Phase mit Wasser, Trocknen über Natriumsulfat und Abdampfen des Lösungsmittels erhält man 1-[2-(4-Carbamoyl-3-hydroxy-phenoxy)-1-methyl-äthylamino]-3-(4-methansulfonylamino-phenoxy)-2-propanol als Diastereomerengemisch, welches aus wenig heissem Isopropanol umkristallisiert wird; Smp. 147-150°.

Das als Ausgangsmaterial benötigte 1-(4-Methansulfonylamino-phenoxy)-3-amino-2-propanol wird wie folgt hergestellt:

8a) Ein Gemisch von 18,7 g 4-Methansulfonylamino-phenol, 8,5 g Epichlorhydrin, 7 g Kaliumcarbonat und 70 ml Isopropanol wird während 2,5 Stunden bei 90° gerührt. Das Reaktionsgemisch wird heiss filtriert, der Rückstand mit Isopropanol gewaschen und das Filtrat unter vermindertem Druck, schliesslich bei 0,03 Torr auf dem Wasserbad eingeengt, wonach man als Rückstand das 4-Methansulfonylamino-(2,3-epoxypropoxy)-phenol erhält.

Eine Lösung von 24,3 g des erhaltenen Produkts in 170 ml Dioxan wird mit einer Lösung von ca. 2 g trocknem Ammoniakgas in 110 ml Dioxan versetzt und das Reaktionsgemisch im verschlossenen Kolben während 24 Stunden bei Raumtemperatur stehen gelassen. Nach Entfernen des überschüssigen Ammoniaks und Abdampfen des Lösungsmittels unter vermindertem Druck erhält man rohes 1-(4-Methansulfonylaminophenoxy)-3-amino-2-propanol, welches als solches weiterverarbeitet wird.

Beispiel 9: 23,2 g 1-[2-(4-Methoxycarbonyl-3-hydroxy-phenoxy)-1-methyl-äthylamino]-3-(4-methansulfonylamino-phenoxy)-2-propanol wird mit einem Gemisch von 50 ml Dioxan und 500 ml konz. Ammoniak-Lösung versetzt. Das Reaktionsgemisch wird 1-2 Stunden gerührt und, sobald es homogen geworden ist, noch 3 Tage bei 20-30° stehen gelassen. Durch Eindampfen erhält man rohes 1-(4-Carbamoyl-3-hydroxy-phenoxy)-3-[2-(4-methansulfonylamino-phenoxy)-1-methyl-äthylamino]-2-propanol als Diastereomerengemisch. Dieses wird in wenig heissem Isopropanol gelöst und die Lösung dann abgekühlt, wobei man die Verbindung rein erhält, Smp. 147-150°.

Das Ausgangsmaterial kann auf folgende Weise erhalten werden:

9a) Eine Lösung von 24,3 g 4-(2,3-Epoxy-propoxy)-methansulfonylamino-phenol und 30 g 4-(2-Benzylamino-propoxy)-methoxycarbonyl-3-hydroxybenzol in 200 ml Isopropanol wird 24 Stunden unter Rückfluss erhitzt. Das nach dem Eindampfen erhaltene Rohprodukt wird zwischen 2-n. Salzsäure und Aether verteilt, die wässrige Phase abgetrennt und mit konz. Ammoniaklösung alkalisch gestellt. Durch erschöpfendes Extrahieren mit Aethylacetat, Waschen der organischen Phase mit Wasser, Trocknen über Magnesiumsulfat und Eindampfen erhält man rohes 1-[N-[2-(4-Carbomethoxy-3-hydroxy-phenoxy)-1-methyl-äthyl]-benzylamino]-3-(4-methansulfonylamino-phenoxy)-2-propanol, welches als solches in der nächsten Stufe weiterverarbeitet wird.

9b) Eine Lösung von 46 g 1-[N-[2-(4-Methoxycarbonyl-3-hydroxy-phenoxy)-1-methyläthyl]-benzylamino]-3-(4-methansulfonylamino-phenoxy)-2-propanol in 500 ml Methanol wird unter Zusatz von 5 g Palladium-auf-Kohle-Katalysator (5%-ig) unter Normalbedingungen bis zur Aufnahme von 1 Mol-Aequivalent Wasserstoff hydriert. Die Lösung wird vom Katalysator abfiltriert und das Filtrat zur Trockne eingeengt, wonach man rohes 1-[2-(4-Methoxycarbonyl-3-hydroxy-phenoxy)-1-methyl-äthylamino]-3-(4-methansulfonylamino-phenoxy)-2-propanol erhält, welches als solches weiterverarbeitet wird.

Beispiel 10: Eine Lösung von 3,7 g 1-[2-(3-Cyano-4-hydroxy-phenoxy)-äthylamino]-3-[4-methansulfonylamino-phenoxy]-2-propanol in einem Gemisch von 15 ml konz. Salzsäure und 25 ml Dioxan wird 15 Stunden bei einer Temperatur von 20-25° gerührt. Das Reaktionsgemisch wird hierauf eingedampft und mit 10%-iger, wässriger Ammoniaklösung alkalisch gestellt. Das nach einigen Stunden Stehen abgeschiedene rohe 1-[2-(3-Carbamoyl-4-hydroxy-phenoxy)-äthylamino]-3-[4-methansulfonylamino-phenoxy]-2-propanol wird abfiltriert und aus Aethanol, dann aus Methanol umkristallisiert; Smp. 152-153°.

Das als Ausgangsmaterial benötigte 1-[2-(3-Cyano-4-hydroxy-phenoxy)-äthylamino]-3-[4-methansulfonylamino-phenoxy]-2-propanol kann wie folgt erhalten werden:

10a) Ein Gemisch von 6,5 g 5-(2-Bromäthoxy)-2-hydroxy-benzonitril

und 9,7 g des nach Beispiel 8a) erhaltenen 1-(4-Methansulfonylamino-phenoxy)-3-amino-2-propanol wird im Oelbad bei 100° geschmolzen und während 1 Stunde magnetisch gerührt. Die Schmelze wird hierauf mit 100 ml Isopropanol ausgekocht, die Lösung filtriert und das Filtrat eingedampft. Der Eindampfrückstand wird zwischen 400 ml Aethylacetat und 50 ml 2-n. Kaliumbicarbonatlösung verteilt, die organische Phase abgetrennt, mit Wasser gewaschen, über Magnesiumsulfat getrocknet und eingedampft, wonach man rohes 1-[2-(3-Cyano-4-hydroxy-phenoxy)-äthyl-amino]-3-[4-methansulfonylamino-phenoxy]-2-propanol erhält, welches als solches weiterverarbeitet wird.

Beispiel 11: 2,3 g Natriummetall werden mit 45 ml Methanol umgesetzt. Nach beendeter Reaktion wird die Lösung von 19,2 g 4-(2-Cyclopropyl-methoxy-äthyl)-phenol (DE-OS 26 49 605) in 110 ml Methanol zugesetzt und das Gemisch während 1 Stunde auf dem Wasserbad unter Rückfluss erhitzt. Anschliessend wird das Reaktionsgemisch unter vermindertem Druck zur Trockne eingedampft und das erhaltene Natriumsalz in 110 ml Dimethylformamid aufgeschlämmt. Dazu gibt man eine Lösung von 28,85 g 5-[N-(3-Chlor-2-hydroxy-propyl)]-aminoäthoxysalicylamid in 170 ml Dimethylformamid, und erwärmt das Gemisch während 3 Stunden unter Rühren auf dem siedenden Wasserbad. Das Lösungsmittel wird unter vermindertem Druck entfernt und der Rückstand mit 200 ml Wasser behandelt, abfiltriert und der Filterrückstand aus Isopropanol, dann aus Methanol umkristallisiert, wonach man reines 1-[2-(3-Carbamoyl-4-hydroxy-phenoxy)-äthylamino]-3-[4-[2-(Cyclopropylmethoxy)-äthyl]-phenoxy]-2-propanol vom Smp. 149-150° erhält.

Das als Ausgangsmaterial benötigte 5-[N-(3-Chlor-2-hydroxy-propyl)]-aminoäthoxy-salicylamid kann wie folgt hergestellt werden:

11a) Ein Gemisch von 48,2 g 2,3-Dihydro-2,2-dimethyl-6-hydroxy-4H-1,3-benzoxazin-4-on, 70 g Kaliumcarbonat und 250 ml 1,2-Dibromäthan wird unter Rühren und Rückfluss während 4 Stunden gekocht. Das breiartige Reaktionsgemisch wird 3-4 mal mit je 1 Liter Methanol heiss extrahiert, die vereinigten Methanol-Extrakte eingedampft und der Rückstand aus Methanol umkristallisiert. Man erhält das 6-(2-Bromäthoxy-2,3-dihydro-2,2-dimethyl-4H-1,3-benzoxazin-4-on vom Smp. 190-195.

11b) Ein Gemisch von 60 g 6-(2-Bromäthoxy)-2,3-dihydro-2,2-dimethyl-4H-1,3-benzoxazin-4-on und 110 ml Benzylamin wird in einem Bad von 80° während 30 Minuten gerührt. Das Reaktionsgemisch wird hierauf unter Eiskühlung mit konz. Salzsäure auf pH 3-4 gebracht und kristallisieren gelassen. Nach 2-4 Stunden werden die Kristalle abgesaugt, mit je 50 ml Wasser und Aethylacetat gewaschen und getrocknet. Das so erhaltene 5-(2-Benzylamino-äthoxy)-salicylamidhydrochlorid schmilzt bei 214-216°. Die daraus freigesetzte Base schmilzt bei 107-108° (aus Aethylacetat-Aether).

11c) Das 5-(2-Aminoäthoxy)-salicylamid kann durch Debenzylierung mittels Wasserstoff in Gegenwart eines Pd/C-Katalysators (5%-ig) der entsprechenden nach Beispiel 11b) erhaltenen N-Benzyl-Verbindung in Methanol hergestellt werden: Smp. 140°.

11d) Eine Lösung von 19,6 g 5-(2-Aminoäthoxy)-salicylamid in 80 ml Methanol wird langsam zu einer Lösung von 9,25 g Epichlorhydrin in 30 ml Methanol zugegeben und das Reaktionsgemisch während 3 Stunden bei 25° gerührt. Nach dem Abdampfen des Lösungsmittels erhält man rohes 5-[N-(3-Chlor-2-hydroxy-propyl)]-aminoäthoxy-salicylamid, welches als solches weiterverarbeitet wird.

Beispiel 12: Zu einem Gemisch von 19,2 g 4-(2-Cyclopropylmethoxyäthyl)-phenol und 25,2 g 1-[2-(3-Carbamoyl-4-hydroxy-phenoxy)-äthyl]-3-azetidinol fügt man 0,4 g festes Kaliumhydroxid und erhitzt das Gemisch während 22 Stunden auf 150° in einer Stickstoffatmosphäre. Nach dem Abkühlen wird das Reaktionsgemisch mit 2-n. Salzsäure neutral eingestellt, mit 100 ml Aether extrahiert, die Aetherphase abgetrennt

und mit 2-n Salzsäure extrahiert. Die wässrige Phase wird abgetrennt, mit konz. Natriumhydroxidlösung neutral eingestellt und das Gemisch mit Methylenchlorid extrahiert. Nach dem Waschen der organischen Phase mit Wasser und Abdampfen des Lösungsmittels wird der erhaltene Rückstand aus Isopropanol, dann aus Methanol umkristallisiert, wonach man das 1-[2-(3-Carbamoyl-4-hydroxy-phenoxy)-äthylamino]-3-[4-[2-(cyclopropylmethoxy)-äthyl]-phenoxy]-2-propanol erhält; Smp. 149-150°.

Das als Ausgangsmaterial benötigte 1-[2-(3-Carbamoyl-4-hydroxy-phenoxy)-äthyl]-3-azetidinol kann auf folgende Weise erhalten werden:

12a) Zu einer auf 80° erwärmten Lösung von 40 g 5-(2-Aminoäthoxy)-2-benzyloxy-benzamid in 250 ml 80%igem Aethanol lässt man unter Rühren innerhalb 1 Stunde eine Lösung von 15,4 g 2-Benzyloxy-1,3-dibrom-propan in 95%-igem Aethanol zutropfen und setzt das Rühren bei der angegebenen Temperatur noch 12 Stunden fort. Anschliessend wird das Lösungsmittel weitgehend entfernt, der Rückstand mit 2-n. Natriumhydroxidlösung alkalisch eingestellt und das Gemisch mit Aethylacetat extrahiert. Die Aethylacetatlösung wird mit 2-n.Salzsäure bis zur sauren Reaktion extrahiert, die organische Phase mit Wasser gewaschen, über Magnesiumsulfat getrocknet und zur Trockne abgedampft, wonach man rohes 1-[2-(3-Carbamoyl-4-benzyloxy-phenoxy)-äthylamino]-3-benzylazetidinol erhält.

Durch Hydrogenolyse mittels Wasserstoff in Gegenwart eines Palladium-auf-Kohle-Katalysators in methanolischer Lösung bis zur Aufnahme von 2 Mol-Aequivalent Wasserstoff erhält man aus der genannten Verbindung das 1-[2-(3-Carbamoyl-4-hydroxy-phenoxy)-äthylamino]-3-azetidinol, welches als solches weiterverarbeitet wird.

<u>Beispiel 13:</u> Tabletten enthaltend 20 mg an aktiver Substanz werden in folgender Zusammensetzung in üblicher Weise hergestellt:

Zusammensetzung:

| | |
|---|---|
| 1-[2-(3-Carbamoyl-4-hydroxy-phenoxy)-äthylamino]-3-[4-[2-(cyclopropylmethoxy)-äthyl]-phenoxy]-2-propanol | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung: 1-[2-(3-Carbamoyl-4-hydroxy-phenoxy)-äthylamino]-3-[4-[2-(cyclopropylmethoxy)-äthyl]-phenoxy]-2-propanol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restlichen Weizenstärke, Talk und Magnesiumstearat zugemischt und die Mischung zu Tabletten von 145 mg Gewicht mit Bruchkerbe verpresst.

Beispiel 14: Tabletten enthaltend 1 mg an aktiver Substanz werden in folgender Zusammensetzung in üblicher Weise hergestellt:

Zusammensetzung:

| | |
|---|---|
| 1-[2-(4-Carbamoyl-3-hydroxy-phenoxy)-1-methyl-äthylamino]-3-(4-methansulfonylamino-phenoxy)-2-propanol | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung: 1-[2-(4-Carbamoyl-3-hydroxy-phenoxy)-1-methyl-äthylamino]-3-(4-methansulfonylamino-phenoxy)-2-propanol wird mit einem Teil der Weizenstärke, mit Milchzucker und kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

Beispiel 15: Kapseln enthaltend 10 mg an aktiver Substanz werden wie folgt auf übliche Weise hergestellt:

Zusammensetzung: 1-[2-(3-Carbamoyl-4-hydroxy-phenoxy)-äthylamino]-3-[2-(2,3-dihydroxypropyl)-phenoxy]-2-propanol 2500 mg

| | |
|---|---|
| Talkum | 200 mg |
| Kolloidale Kieselsäure | 50 mg |

Herstellung: Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

Beispiel 16: Eine sterile Lösung von 5,0g 1-[2-(3-Carbamoyl-4-hydroxy-phenoxy)-äthylamino]-3-[2-(2,3-dihydroxypropyl)-phenoxy]-2-propanol-hydrochlorid in 5000 ml destilliertem Wasser wird in Ampullen zu 5 ml abgefüllt, die in 5 ml Lösung 5 mg Wirkstoff enthalten.

Beispiel 17: Anstelle der in den Beispielen 13 bis 16 als aktive Substanzen verwendeten Substanzen können auch folgende Verbindungen der Formel I oder deren pharmazeutisch verwendbare nicht-toxischen Säureadditionssalzen als aktive Substanzen in Tabletten, Dragées, Kapseln, Ampullenlösungen etc. verwendet werden: 1-[2-(3-Carbamoyl-4-hydroxy-phenoxy)-äthylamino]-3-(4-methylsulfonylamino)-phenoxy-2-propanol, 4-[3-[2-(3-Carbamoyl-4-hydroxy-phenoxy)-äthylamino]-2-hydroxy-propoxy]-N-methyl-zimtsäureamid oder das 1-[2-(3-Carbamoyl-4-hydroxy-phenoxy)-äthylamino]-3-[4-(N-methyl-methansulfonylamino)-phenoxy]-2-propanol.

Patentansprüche:

1. Derivate des 3-Amino-1,2-propandiols der Formel

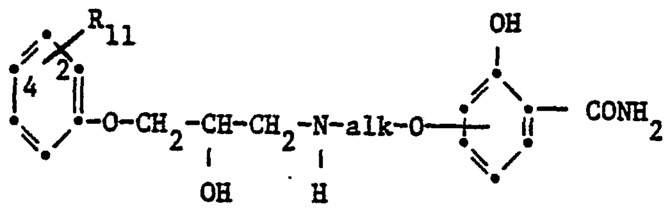

(I)

in welcher $R_{11}$ Polyhydroxyniederalkyl, Cycloalkylniederalkoxyniederalkyl, in 4-Stellung gebundenes Niederalkylsulfonylamino oder in 4-Stellung gebundenes 1-Niederalkylcarbamoyl-2-vinyl, und alk Reste der Formeln $-CH_2CH_2-$ oder $-CH(CH_3)-CH_2-$ bedeutet.

2. Verbindungen der Formel I, worin $R_{11}$ Di- oder Trihydroxyniederalkyl, Cycloalkylniederalkoxyniederalkyl mit bis zu 4 Kohlenstoffatomen im Niederalkylteil, in 4-Stellung gebundenes Niederalkyl- oder N-Niederalkyl-niederalkylsulfonylamino mit bis zu 4 Kohlenstoffatomen in jedem Niederalkylteil, oder in 4-Stellung gebundenes 1-Niederalkylcarbamoyl-2-vinyl mit bis zu 4 Kohlenstoffatomen im Niederalkylteil, und alk Reste der Formeln $-CH_2-CH_2-$ oder $-CH(CH_3)-CH_2-$ bedeutet.

3. Verbindungen der Formel I, worin $R_{11}$ 1,2-Dihydroxy- oder 2,3-Dihydroxyniederalkyl, Cycloalkylniederalkoxymethyl oder 1- oder 2-(Cycloalkylniederalkoxy)-äthyl, in 4-Stellung gebundenes Niederalkylsulfonylamino mit bis zu 4 Kohlenstoffatomen, wobei $R_{12}$ Wasserstoff ist, oder in 4-Stellung gebundenes 1-Aethylcarbamoyl-2-vinyl oder 1-Methylcarbamoyl-2-vinyl, und alk Reste der Formeln $-CH_2-CH_2-$ oder $-CH(CH_3)-CH_2-$ bedeutet.

4. Verbindungen der Formel I, worin $R_{11}$ 1,2-Dihydroxy- oder 2,3-Dihydroxyniederalkyl mit bis zu 4 Kohlenstoffatomen, z.B. 1,2-Dihydroxy- oder 2,3-Dihydroxypropyl, 1- oder 2-(Cycloalkylniederalkoxy)-äthyl mit 3-5 Kohlenstoffatomen im Cycloalkylteil, z.B. 1- oder 2-(Cyclopropylmethoxy)-äthyl, oder 1-Methylcarbamoyl-2-vinyl, und alk Reste der Formeln $-CH_2-CH_2-$ oder $\overset{\displaystyle CH_3}{\overset{|}{-CH}}-CH_2-$ bedeutet.

5. 1-[2-(3-Carbamoyl-4-hydroxy-phenoxy)-äthylamino]-3-[2-(2,3-dihydroxypropyl)-phenoxy]-2-propanol.

6. 1-[2-(4-Carbamoyl-3-hydroxy-phenoxy)-1-methyl-äthylamino]-3-(4-methansulfonylaminophenoxy)-2-propanol.

7. 1-[2-(3-Carbamoyl-4-hydroxy-phenoxy)-äthylamino]-3-[4-[2-(cyclopropylmethoxy)-äthyl]-phenoxy]-2-propanol.

8. 1-[2-(3-Carbamoyl-4-hydroxy-phenoxy)-äthylamino]-3-(4-methansulfonylaminophenoxy)-2-propanol.

9. 4-[3-[2-(3-Carbamoyl-4-hydroxy-phenoxy)-äthylamino]-2-hydroxypropoxy]-N-methyl-zimtsäureamid.

10. Salze von Verbindungen der Ansprüche 1-9.

11. Pharmazeutisch verwendbare, nicht-toxische Säureadditionssalze von Verbindungen der Ansprüche 1-9.

12. Pharmazeutische Präparate enthaltend eine Verbindung der Formel I.

13. Verbindungen der Formel I zur Anwendung in einem Verfahren zur therapeutischen Behandlung des menschlichen oder tierischen Körpers.

14. Verbindungen der Formel I als Mittel zur Bekämpfung von Angina pectoris, Herzrythmusstörungen und des Bluthochdrucks.

15. Verwendung von Verbindungen der Formel I zur Herstellung von pharmazeutischen Präparaten.

16. Verfahren zur Herstellung von Derivaten des 3-Amino-1,2-propandiols der Formel

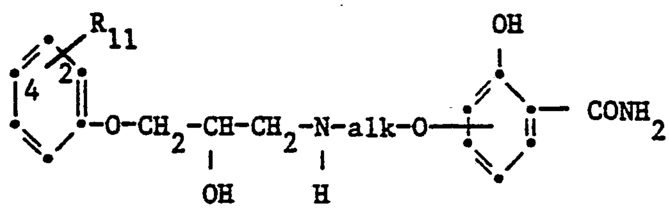

(I)

in welcher $R_{11}$ Polyhydroxyniederalkyl, Cycloalkylniederalkoxyniederalkyl, in 4-Stellung gebundenes Niederalkylsulfonylamino oder in 4-Stellung gebundenes 1-Niederalkylcarbamoyl-2-vinyl und alk Reste der Formeln $-CH_2-CH_2-$ oder $-CH(CH_3)-CH_2$ bedeutet, dadurch gekennzeichnet,

dass man

a) eine Verbindung der Formel

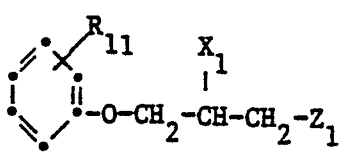

(II)

mit einer Verbindung der Formel

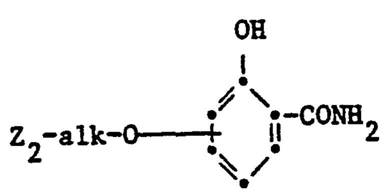

(III)

worin eine der Gruppen $Z_1$ und $Z_2$ eine reaktionsfähige veresterte Hydroxygruppe darstellt und die andere für die primäre Aminogruppe steht, und $X_1$ Hydroxy bedeutet, oder worin $X_1$ und $Z_1$ zusammen die Epoxygruppe bedeuten und $Z_2$ für die primäre Aminogruppe steht, und $R_{11}$ und alk obige Bedeutung haben, umsetzt, oder

b) in einer Verbindung der Formel

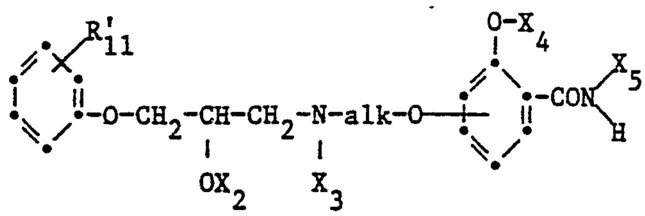

(IV),

worin $R'_{11}$ die Bedeutung von $R_{11}$ hat, oder in 4-Stellung gebundenes, am Stickstoffatom durch einen durch Wasserstoff ersetzbaren Substituenten substituiertes Niederalkylsulfonylamino darstellt, $X_2$, $X_3$, $X_4$ und $X_5$ jeweils Wasserstoff oder einen durch Wasserstoff ersetzbaren Substituenten bedeuten oder $X_2$ und $X_3$ und/oder $X_4$ und $X_5$ zusammen einen zweiwertigen, durch zwei Wasserstoffatome ersetzbaren Rest bedeuten, mit der Massgabe, dass mindestens einer der Reste $X_2$, $X_3$, $X_4$ und $X_5$ von Wasserstoff verschieden ist, oder mindestens $R'_{11}$ in 4-Stellung gebundenes, am Stickstoffatom, durch einen durch Wasserstoff ersetzbaren Substituenten substituiertes Niederalkylsulfonylamino darstellt, oder mindestens $X_2$ und $X_3$ zusammen oder $X_4$ und $X_5$ zusammen einen zweiwertigen, durch zwei Wasserstoffatome ersetzbaren Rest darstellen, oder in einem Salz davon, die von Wasserstoff verschiedenen Gruppen $X_2$, $X_3$, $X_4$ und $X_5$ bzw. $X_2$ und $X_3$ zusammen und/oder $X_4$ und $X_5$ zusammen abspaltet und durch Wasserstoffatome ersetzt, und/oder einen am Stickstoffatom von in 4-Stellung gebundenem Niederalkylsulfonylamino $R'_{11}$ stehenden durch Wasserstoff ersetzbaren Substituenten durch Wasserstoff ersetzt, oder,

c) in einer Verbindung der Formel

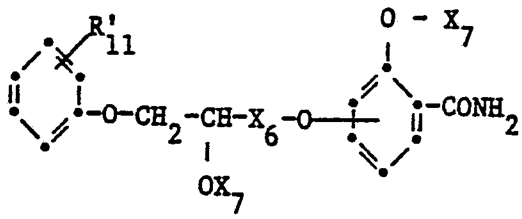

(V),

worin $X_6$ eine reduzierbare Gruppe der Formeln

$-CH = N - alk -$ (Va), bzw.

$-CH_2 - N = alk_1 -$ (Vb)

ist, wobei $alk_1$ für einen, dem Rest alk entsprechenden Alkylylidenrest steht und $X_7$ Wasserstoff oder einen unter den Bedingungen für die Reduktion von $X_6$ durch Wasserstoff ersetzbaren Rest darstellt, $R'_{11}$ die Bedeutung von $R_{11}$ hat, oder in 4-Stellung gebundenes, am Stickstoffatom durch einen durch Wasserstoff ersetzbaren Substituenten $X_7$ substituiertes Niederalkylsulfonylamino darstellt, worin $X_7$ die vorstehend angegebene Bedeutung hat, wobei stets $X_6$ eine reduzierbare Gruppe Va oder Vb ist, diese Gruppe reduziert und gleichzeitig die von Wasserstoff verschiedene Gruppe $X_7$ abspaltet und durch Wasserstoff ersetzt, oder

d) eine Verbindung der Formel

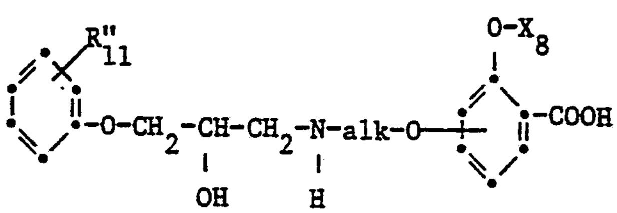

(VI),

worin $R''_{11}$ die Bedeutung von $R_{11}$ hat, oder in 4-Stellung gebundenes, am Stickstoffatom durch einen mittels Ammonolyse durch einen durch Wasserstoff ersetzbaren Substituenten substituiertes Niederalkylsulfonyl-

amino darstellt, $X_8$ Wasserstoff oder eine mittels Ammonolyse abspaltbare Gruppe bedeutet, oder ein reaktionsfähiges Derivat einer der in Formel VI definierten Carbonsäure, mit Ammoniak (VII) umsetzt, und gleichzeitig gegebenenfalls vorhandene Reste $X_8$ abspaltet und durch Wasserstoff ersetzt, oder

e) in einer Verbindung der Formel

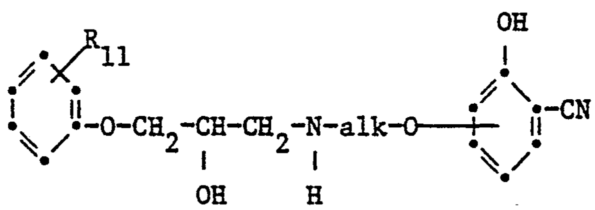

(VII),

worin eine oder alle Hydroxygruppen und/oder das sekundäre Stickstoffatom und/oder in 4-Stellung gebundenes Niederalkylsulfonylamino $R_{11}$ am Stickstoffatom gegebenenfalls durch solche mittels Hydrolyse abspaltbaren und durch Wasserstoff ersetzbaren Gruppen substituiert sind, die unter den Bedingungen des Verfahrens abgespalten und durch Wasserstoff ersetzt werden, die Gruppe -CN mittels Hydrolyse in die Gruppe $-CONH_2$ umwandelt, und gleichzeitig gegebenenfalls am Stickstoffatom einer in 4-Stellung gebundenen Niederalkylsulfonylaminogruppe und/oder an einer oder allen Hydroxygruppen und/oder am sekundären Stickstoffatom stehende Schutzgruppen abspaltet, und durch Wasserstoff ersetzt, oder

f) zur Herstellung einer Verbindung der Formel I, worin $R_{11}$ Polyhydroxyniederalkyl bedeutet, in einer Verbindung der Formel

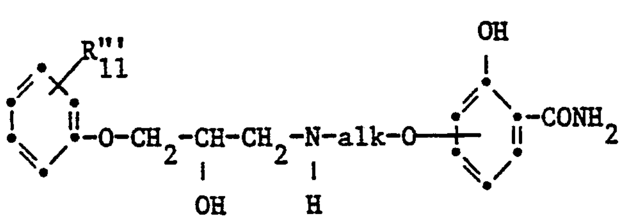

(VIII)

worin $R_{11}'''$ eine Polyhydroxyniederalkylgruppe, z.B. eine der genannten, bedeutet, worin mindestens eine, oder zwei Hydroxygruppen zusammen, wovon je eine an zwei benachbarten Kohlenstoffatomen steht, durch einen abspaltbaren und durch Wasserstoff ersetzbaren Rest geschützt ist, oder in einem Salz davon, diese Schutzgruppen, die gleich oder verschieden sein können, sowie gegebenenfalls weitere am Stickstoffatom und/oder an den Sauerstoffatomen stehende Schutzgruppen abspaltet und durch Wasserstoff ersetzt, oder

g) zur Herstellung einer Verbindung der Formel I, worin $R_{11}$ eine in 4-Stellung gebundene 4-Niederalkylsulfonylaminogruppe darstellt, in eine Verbindung der Formel

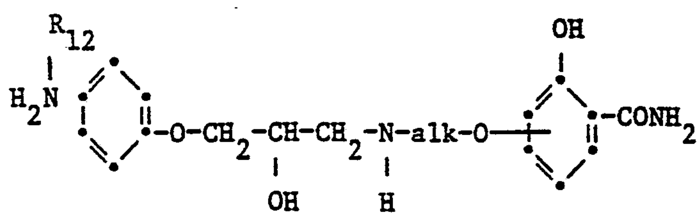

(IX) ,

worin $R_{12}$ Wasserstoff oder Niederalkyl bedeutet, die Niederalkylsulfonylgruppe einführt, oder

h) eine Verbindung der Formel

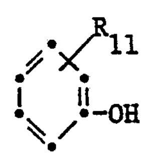

(X)

mit einer Verbindung der Formel

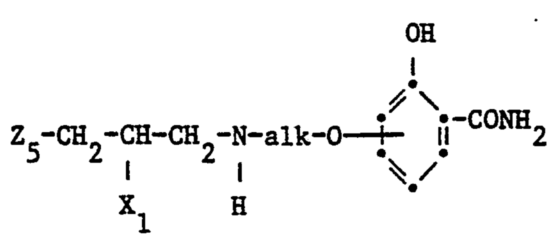

(XI),

worin $Z_5$ für eine reaktionsfähige, veresterte Hydroxygruppe steht und $X_1$ Hydroxy ist, oder worin $Z_5$ und $X_1$ zusammen die Epoxygruppe bedeuten, oder mit einer entsprechenden ringgeschlossenen Verbindung der Formel

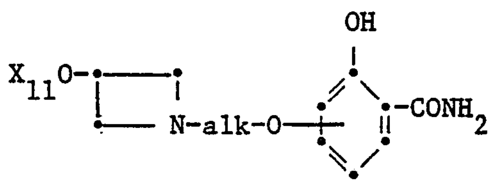

(XII),

worin $R_{11}$ und alk jeweils die angegebene Bedeutung haben, und $X_{11}$ für Wasserstoff oder eine unter den Bedingungen des Verfahrens abspaltbare und mit der Hydroxygruppe einer Verbindung der Formel X zur Bildung der Aetherbindung befähigte Gruppe steht, umsetzt, und, wenn erwünscht, eine erhaltene freie Verbindung in ein Salz oder ein erhaltenes Salz in eine freie Verbindung überführt, und/oder, wenn erwünscht, ein erhaltenes Isomerengemisch in die Isomeren oder ein erhaltenes Racemat in die Antipoden auftrennt.

17. Die nach dem Verfahren des Anspruchs 16 erhältlichen Verbindungen.

0045964

Europäisches Patentamt

# EUROPÄISCHER RECHERCHENBERICHT

Nummer der Anmeldung

EP 81 10 6233.0

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| P | EP - A1 - 0 015 505 (CIBA-GEIGY)<br>* Ansprüche 1, 4, 5 *<br>-- | 1 | C 07 C 103/26<br>C 07 C 143/75<br>C 07 C 103/58<br>A 61 K 31/165<br>A 61 K 31/18 |
| A | EP - A1 - 0 005 848 (CIBA-GEIGY)<br>-- | | |
| A | DE - A - 2 357 849 (PFIZER CORP.)<br>---- | | |
| | | | RECHERCHIERTE SACHGEBIETE (Int. Cl.³) |
| | | | A 61 K 31/165<br>A 61 K 31/18<br>C 07 C 103/26<br>C 07 C 103/58<br>C 07 C 143/75 |
| | | | KATEGORIE DER GENANNTEN DOKUMENTE |
| | | | X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 30-10-1981 | PHILLIPS |